# EUROPEAN PATENT APPLICATION

(11) **EP 4 620 523 A1**
(43) Date of publication of application: **24.09.2025**
(21) Application number: 24382313.5
(22) Date of filing: 22.03.2024
(51) Int. Cl.: A61P 9/00, A61P 11/00, A61P 13/12, A61P 21/00, A61P 25/00, A61P 35/00, A61P 37/00, C07K 14/72, C07K 16/28

(54) **MUTANTS OF THE BETA2-ADRENERGIC RECEPTOR WITH ALTERED ACTIVITY AND THEIR USE IN DISEASE TREATMENT.**

(71) Applicant: Genome Research Limited, Hinxton, Saffron Walden CB10 1SA (GB); Fundació Centre de Regulació Genòmica, 08003 Barcelona (ES)
(72) Inventor: MIGHELL, Taylor, Barcelona (ES); LEHNER, Benjamin, Hinxton (GB)
(74) Representative: D Young & Co LLP

(57) **Abstract**

The present invention relates to a method of modulating the activity of a β2-adrenergic receptor (β2AR), the method comprising the step of mutating one or more residues identified as being allosteric. The invention also relates to a binding molecule which binds to one or more identified allosteric residues, a polypeptide encoding a β2-adrenergic receptor (β₂AR) variant mutated at one or more identified allosteric residues and use of these in therapy.

## Description

### FIELD OF THE INVENTION

The present invention relates to methods for modulating the activity of a β₂-adrenergic receptor (β₂AR), the methods comprising mutating β₂AR or providing a binding molecule which binds to β₂AR. In particular, the methods comprise targeting allosteric sites on β₂AR in order to modulate its activity. The invention also encompasses said binding molecules, modified β₂ARs, and methods for treating diseases associated with β₂AR.

### BACKGROUND OF THE INVENTION

G-protein coupled receptors (GPCRs) are integral transmembrane proteins, which receive signals from the extracellular environment and transmit them across the lipid bilayer.

GPCRs constitute a large class of structurally and functionally related molecules. GPCRs prototypically comprise 7 membrane-spanning helices, which form both extra-cellular and intracellular loops capable of interacting with ligands. The transmembrane portion of the GPCR is associated with one or more G-proteins, which each comprise α-, β-, and γ-subunits, on the intracellular side of the receptor.

GPCRs mediate the perception of diverse signals, which include chemical signals such as neurotransmitters and immune system molecules, as well as photons. Typically, binding of a GPCR ligand molecule to the extracellular portion of the receptor results in a conformational change of the receptor that is translated through the receptor, resulting in a conformational change of the intracellular portion of the receptor. This conformational change results in the release of GDP from the α-subunit of the associated G-protein(s), enabling binding of GTP and activation of the G-protein(s). This causes the α-subunit(s) and bound GTP to dissociate from the GPCR and interact with their relevant effectors resulting in the downstream effects associated with activation of the GPCR.

Activation of GPCRs can result in varied downstream effects depending on the one or more G-proteins coupled to receptor and in particular, the type of α-subunit associated with the G-protein. For example, Gₛ α-subunits stimulate adenylyl cyclase, resulting in an increase in cAMP, whereas G_{i/o} α-subunits inhibit adenylyl cyclase, resulting in a decrease in cAMP. G_{Q/11} α-subunits stimulate phospholipase C, which results in an increase of IP3 and DAG and finally an increase in cytoplasmic calcium levels and other downstream effects. Finally, G_{12/13} α-subunits have been shown to activate the RhoA pathway.

In addition to the prototypic G-proteins that GPCRs activate, GPCRs can also mediate cellular signalling via direct interactions with other molecules, such as β-arrestin. Indeed, a single GPCR may signal down more than one specific signalling pathway, even in response to the same ligand.

The variety of downstream effects means that a number of different methodologies can be used to measure the functional response of GPCRs to different ligands. For example, fluorescent calcium flux assays may be used to monitor the functional response of G_{Q} associated receptors. Alternatively, cAMP assays may be used to monitor the functional response of Gₛ and Gᵢ associated receptors. Other assays, such as GTPγS, β-arrestin and receptor internalization assays may also be used.

Because of their role as master signal transducers and their presence on the cell surface, GPCRs make excellent drug targets, indeed, GPCRs are the target for approximately one third of medicinal drugs.

Despite the wide utility of targeting GPCRs and the relatively well-studied nature of this class of receptors, complexities in GPCR activation and signalling mean that effectively targeting these receptors often remains challenging.

GPCRs are not binary conformational switches; they sample an ensemble of conformations, each of which may have different efficacies for stimulating G-protein dependent or independent pathways (e.g. β-arrestin-mediated). Different ligands can differentially and/or preferentially stabilize certain conformations, which may result in preferential signalling via one pathway; a phenomenon known as biased agonism.

Given the dynamic nature of GPCRs and their varied ligands, it follows that different ligands may actually mediate their effects by engaging different allosteric networks within the GPCR. However, the existence of such allosteric networks remains largely unexplored.

Furthermore, most existing drugs that target GPCRs target an orthosteric site. However, allosteric drugs that bind GPCRs at a distinct site away from said orthosteric site could afford improved specificity.

Hence, the lack of knowledge of allosteric networks within GPCRs has hampered the development of new or improved agents that modulate GPCR activity.

### SUMMARY OF THE INVENTION

The present invention relates to β₂-adrenergic receptor (β₂AR) and the identification, by the present inventors, of allosteric sites on β₂AR that are capable of modulating its activity.

The inventors have identified numerous surface sites within β₂AR that are allosterically active. The present invention relates to said sites.

In one aspect, there is provided a method of modulating the activity of a β₂-adrenergic receptor (β₂AR), the method comprising the step of mutating one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, 1121, L124, V126, 1127, A128, V129, D130, R131, P138, F139, Y141, L145, 1153, V160, S161, T164, S165, F166, P168, Y174, A181, 1182, C184, Y185, N187, C190, C191, F193, Y199, A200, S203, S204, S207, F208, Y209, P211, V218, Y219, E225, Q229, D234, S236, F240, S246, G255, E268, H269, L272, G276, 1277, I278, G280, T283, C285, W286, F289, F290, N293, I294, V295, Y308, N312, W313, 1314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407

In one embodiment, the modulating is activating or inactivating.

In one aspect, there is provided a binding molecule which binds to one or more target sites on a β₂-adrenergic receptor (β₂AR), wherein the one or more target sites comprises one or more residues selected from the group consisting of: N6, P14, N15, S17, P20, V24, Q26, R28, M36, G37, 143, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407

In one embodiment, the one or more target sites is druggable.

In one embodiment, the binding molecule is:
(i) a polypeptide;
(ii) a nucleic acid; or
(iii) a small molecule.

In one embodiment, the binding molecule is an antibody, or a mimetic or derivative thereof, optionally an affibody, a nanobody, a Fab fragment, or a scFv.

In one embodiment, the binding molecule modulates the activity of β₂AR, optionally wherein the modulating is an increase or a decrease in activity.

In one aspect, there is provided a polypeptide encoding a β₂-adrenergic receptor (β₂AR) variant, wherein the polypeptide comprises a mutation, relative to a wild-type β₂AR, at one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

In one aspect, there is provided a nucleic acid encoding:
(i) the binding molecule of any one of claims 3 to 7; and/or
(ii) the polypeptide of claim 8.

In one embodiment, the nucleic acid is RNA or DNA, optionally wherein the nucleic acid is modified, unmodified, naturally occurring or synthetic.

In one aspect, there is provided an expression cassette comprising the nucleic acid as described herein.

In one aspect, there is provided a vector comprising the nucleic acid, or the expression cassette as described herein.

In one aspect, there is provided a cell comprising the binding molecule, the, the nucleic acid, the expression cassette, or the vector as described herein.

In one aspect, the binding molecule, the polypeptide, the nucleic acid, the expression cassette, the vector, or the cell as described herein, is provided for use in a method of treating a disease.

In one embodiment, the disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.

### BRIEF DERCRIPTION OF THE DRAWINGS

Further aspects, features and advantages of the present technique will be apparent from the following description of examples, which is to be read in conjunction with the accompanying drawings, in which:

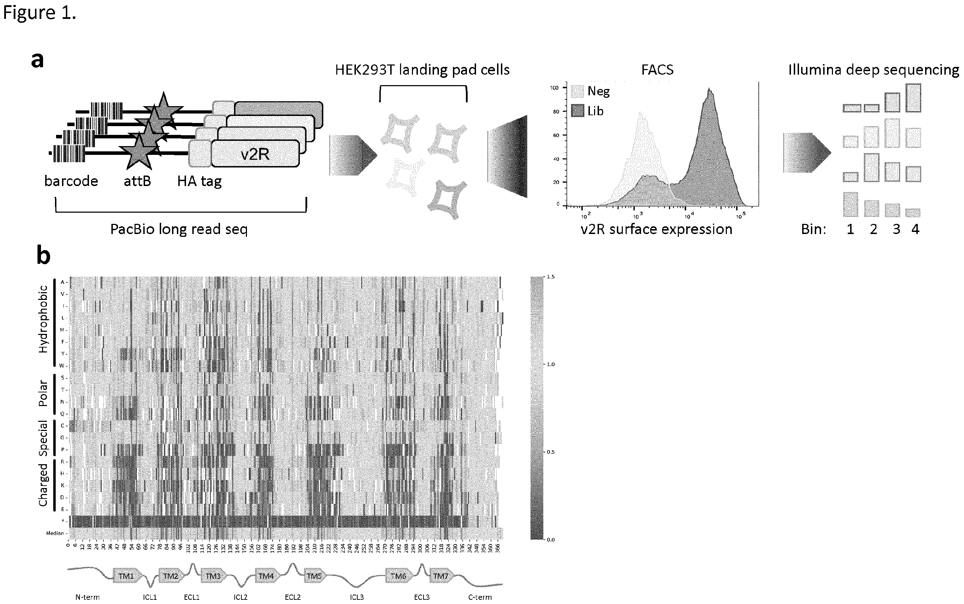

**Figure 1****: overview of surface expression experiment. A.** Schematic of the method. A library of GPCR variants (in this case vasopressin 2 receptor (v2R)) with N-terminal HA tag is expressed in cells. Fluorescent staining followed by FACS followed by Illumina sequencing provides an estimate of surface expression level for each variant. **B.** Heatmap representation of surface expression data for v2R.

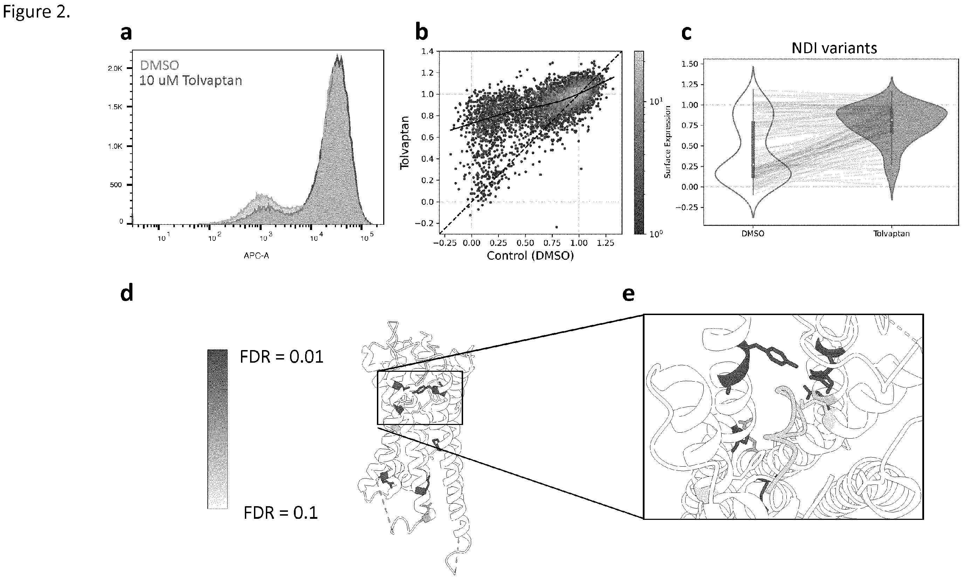

**Figure 2****: Pharmacological rescue of v2R with Tolvaptan. A.** FACS distributions of cells expressing v2R in presence of DMSO (grey, control) or Tolvaptan (pharmacological chaperone, red). **B.** Comparison of all variant surface expression scores in control condition versus Tolvaptan rescue condition. A loess curve has been fit to the data. **C.** Identification of variants, associated with nephrogenic diabetes insipidus, whose surface expression is rescued by Tolvaptan. **D.** Positions with variant scores significantly deviating (i.e. high residuals) from loess fit curve. A residual is calculated for all variants as the distance between the loess curve and the surface expression in the Tolvaptan condition. Then, for each position, a statistical test (for example: Mann-Whitney U test, t-test, Z-test, etc.) compares the residuals of that position with the residuals of variants at all other positions. Alternatively, the magnitude (for example, mean, median, maximum, etc.) of residuals at each position could be compared. In this case, we performed Mann-Whitney U test followed by FDR correction (with 10% FDR correction) with the Benjamini / Hochberg method. Positions with significantly different residuals are highlighted on the structure. **E.** A blown-up view of the putative binding site of Tolvaptan. In purple is the endogenous ligand, vasopressin. We propose this method can be used to map the binding site of molecules with unknown binding site.

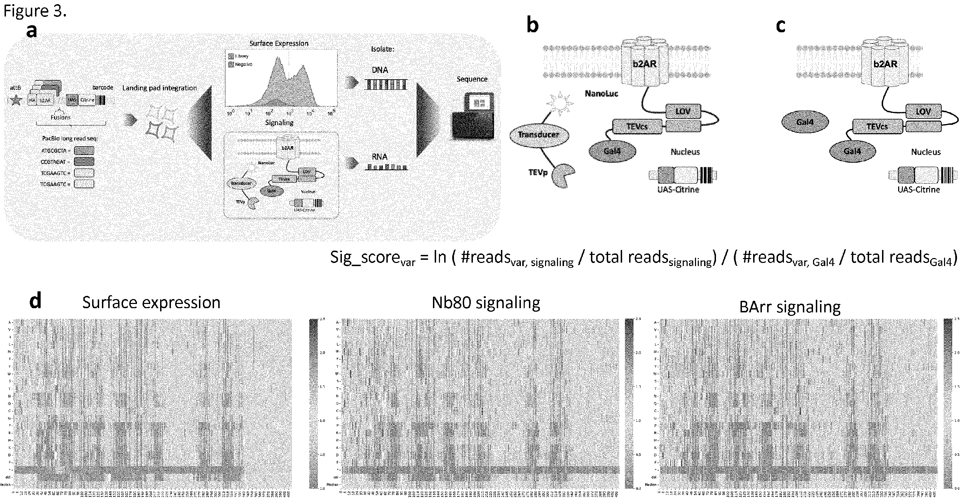

**Figure 3****: Overview of signalling assay A.** Schematic of the method. In order to determine a residue's role in signalling, one must also measure its effect on surface expression. **B.** The assay to measure GPCR signalling. The "Transducer" could be a nanobody, G-protein, miniG protein, arrestin, or anything that binds to the receptor in an activity-dependent manner. Use of the nanobody Nb80, the arrestin B-Arrestin2 (BArr), and miniGₛ393 have been validated. **C.** The normalization assay for GPCR signalling. The receptor construct and the barcode construct are expressed as usual. However, instead of expressing the transducer construct, a free Gal4 is expressed, which should drive maximal expression of the barcode. Any differences in expression level between different barcodes would be due to (i) different abundance in the library or (ii) RNA stability/expression differences intrinsic to the barcode sequence itself. Because both of these factors should also be present in the signalling setup, by normalizing to the Gal4 system, contribution of those factors to measurements should be eliminated. **D.** Heatmap representation of surface expression, signalling through Nb80, and signalling through B-arrestin.

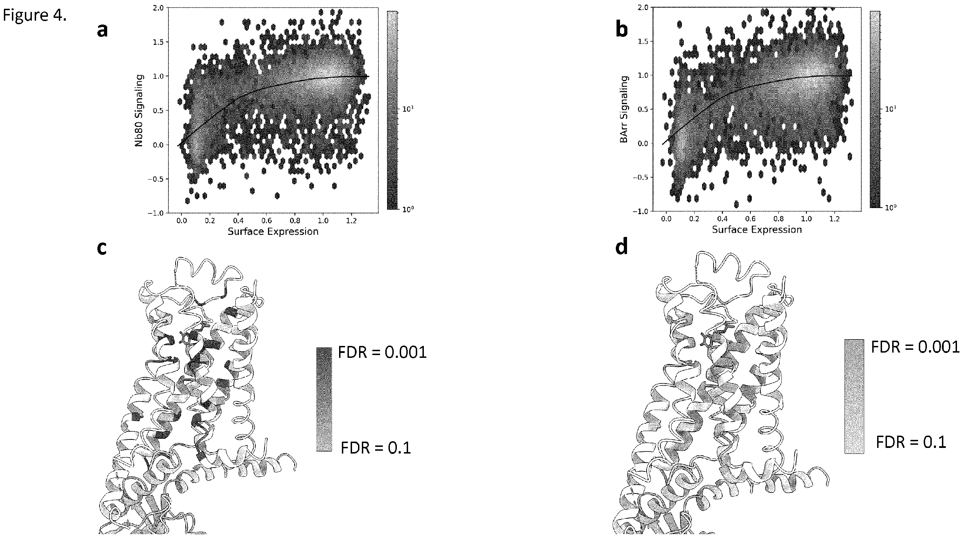

**Figure 4****: Identifying residues important for signalling through both canonical pathways. A.** The Nb80 signalling score of each variant plotted against its surface expression score. A loess curve has been fit to the data. **B.** The BArr signalling score of each variant plotted against its surface expression score. A loess curve has been fit to the data. **C.** A residual is calculated for all variants as the distance between the loess curve and the measured Nb80 signalling score. Then, for each position, a statistical test (for example: Mann-Whitney U test, Z-test, etc.) compares the residuals of that position with the residuals of variants at all other positions. Alternatively, the magnitude (for example, mean, median, maximum, etc.) of residuals at each position could be compared. In this case, we performed Mann-Whitney U test followed by FDR correction with the Benjamini / Hochberg method. **D.** Same as (c) but for BArr signalling.
**Figure 5****: Annotation of residues identified in Nb80 model** To show that the approach works for identifying known functional residues, the fraction of each functional class captured by the model is shown. Here, the Nb80 model is shown because a structure of Nb80-bound β₂AR has been solved. All of the ligand binding residues are identified, while half of residues in known functional motifs are identified. Likewise, many residues in the Nb80 interface or in the second shell of ligand binding or Nb80 binding interface are identified.
**Figure 6****:** illustrates a method for identification of target sites of a GPCR.
**Figure 7****:** illustrates a method for generating a training data set.
**Figure 8****:** illustrates a method for identifying target sites based on residuals between a predicted functional measure and an actual function measure.
**Figure 9****:** illustrates a method for combining more than one functional measure to identify target sites of a GPCR.
**Figure 10****:** illustrates a computer apparatus suitable for implementing methods according to the present disclosure.

### DETAILED DESCRIPTION OF THE INVENTION

The present inventors sought to provide a detailed understanding of the allosteric networks within GPCRs, specifically β₂AR, that link ligand binding to intracellular signalling within these dynamic receptors. Such an understanding enables the design of new or improved drugs that target GPCRs, as well as mutated versions of GPCRs which may have therapeutic and non-therapeutic utility.

The present inventors have employed a multidimensional deep mutational scanning (DMS) approach to measure the functional effects of nearly all single, and thousands of double, amino acid mutations in β₂AR.

The mapping performed by the inventors has identified multiple sites within β₂AR that are allosterically active and are thus able to modulate β₂AR activity. These allosteric sites facilitate the modulation of β₂AR activity for therapeutic and non-therapeutic applications.

Furthermore, the inventors provide a GPCR activity assay that may be used to perform said mapping to identify allosteric sites, as well as tools and reagents for performing said activity assay. Given the shared functionality of GPCRs due to the commonalities in intracellular signalling, the activity assays herein, whilst exemplified using β₂AR, may be applied to other GPCRs and this is encompassed within the scope of the present application.

### Method for identifying target sites

A method is provided for identifying one or more target sites of a G-protein coupled receptor (GPCR).

The method comprises: obtaining a training data set specifying, for each of a plurality of variants of the GPCR having different combinations of one or more mutations, a surface expression measure and a functional measure for that variant, the functional measure quantifying an extent to which the variant is functional for a given purpose; fitting a model to the training data set, to obtain a set of model parameters indicative of a correlation between the surface expression measure and the functional measure for the variants of the GPCR; and based on the set of model parameters obtained by fitting the model, identifying the one or more target sites of the GPCR.

This approach provides a deep mutational scanning (DMS) approach to investigating which sites of a GPCR have an effect on whether the GPCR is functional for a given purpose of interest (e.g. the given purpose could be increased or decreased signalling, increased or decreased surface expression when subject to a ligand, etc.). Mutations are used as a perturbation of the wild type GPCR, to investigate whether a given site has an effect on the functional behaviour of the GPCR, based on whether a mutation at that site causes a change in the functional behaviour. However, in practice, if an experimental assay is performed to obtain a functional measure quantifying the extent to which the mutated variant of the GPCR is functional for the required purpose, it may be difficult to disambiguate whether an increase or decrease of the functional measure for a given mutated variant relative to the wild-type GPCR is due to the site affected by the mutation actually influencing the functional behaviour of the GPCR, or due to the mutation altering the level of surface expression of the GPCR so that in the measured population of samples there is a larger or smaller fraction of that population with surface expressed GPCRs. An increased or decreased surface expression of the GPCR may cause a corresponding change in the measured functional measure (if there are more or fewer samples available to cause the effect being measured as the functional measure), so a functional measure on its own may not give useful information about which sites are the most likely to have an influence on function for reasons other than an increased/decreased level of surface expression. For selecting sites that may be targets for drugs or other treatments, it can be more useful to select sites that have an effect on functional behaviour for reasons other than controlling the level of surface expression.

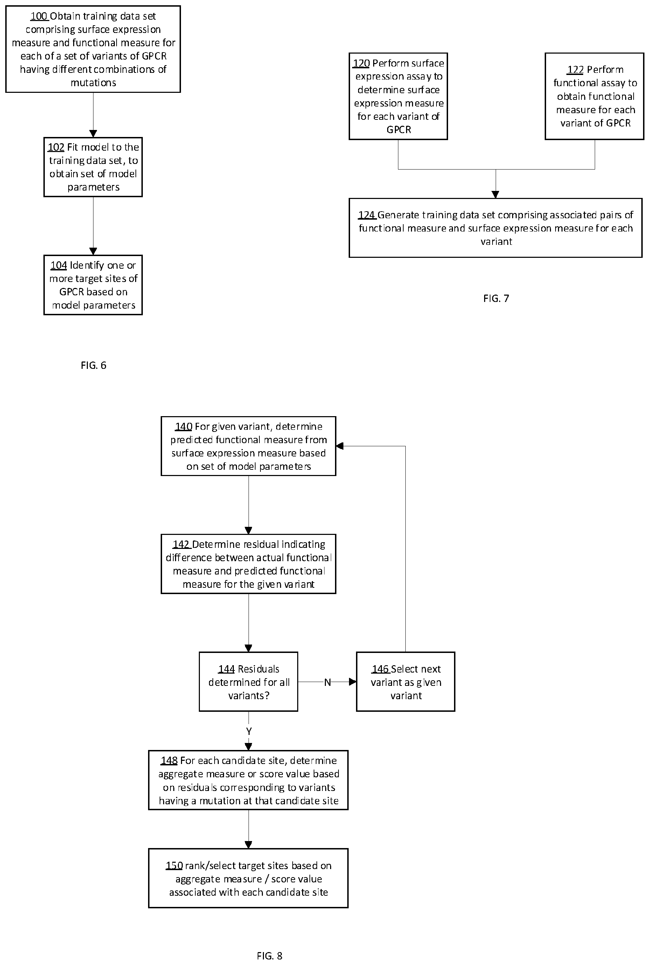

Figure 6 shows a method for identifying target sites of the GPCR. At step 100, a training data set is obtained specifying, for each of a set of variants of GPCR having different combinations of one or more mutations, a surface expression measure and a functional measure for that variant. The training data set can be obtained either by actually performing wet lab experimental work and generating the training data set based on measurements made in the experimental work, or by reading in a previously obtained training data set from a storage medium or over a network interface. The entity performing the steps of Figure 6 may not necessarily be the same as the entity performing the experimental assays for obtaining the measurements used to generate the training data set. The surface expression measure for a given variant is an indication of the level of surface expression associated with the given variant, and the functional measure is a measure of the extent to which the given variant is functional for a required purpose. For example, the functional measure could be a further surface expression measure measured for the given variant in the presence of a ligand, if the functional purpose of interest for the selection of target sites is the extent to which pharmacological rescue of the level of surface expression using a drug or other ligand is possible. Alternatively, the functional measure could be a signalling measure for the given variant, if the purpose of interest for selecting target sites is the extent to which the target site is relevant to controlling signalling across a cellular membrane using the GPCR.

At step 102, the method comprises fitting a model to the training data set, to obtain a set of model parameters indicative of a correlation between the surface expression measure and the functional measure for the variants of the GPCR. For example, the model may be a regression model. The model parameters define a function by which a predicted value for the functional measure can be determined from a corresponding value of the surface expression measure. For example, the model parameters may define a line, curve, polynomial expression, spline, or piecewise function fitted to the training data to enable prediction of the functional measure from the surface expression measure. In some examples, the model parameters may define a non-linear function relating the surface expression measure to the functional measure. For example, the model may be a neural network or other machine learning model that has parameters governing the transformation function for mapping a given input (the surface expression measure) to an output (the predicted functional measure). Regardless of the model type, a training function may be used to adapt the model parameters based on the training examples (pairs of surface expression measure and functional measure for a given mutated variant of the GPCR). The training function may be an error minimisation function such as a least square error or gradient descent method, which minimises a cost function expressing a difference (residual or error) between a predicted functional measure predicted by the model based on surface expression measure and the actual functional measure from the training data set that corresponds to that surface expression measure. The particular training method may depend on the type of model used. Although any regression model may be used, in the example of Figure 4 the model is a Loess model (a particular example of a local regression model).

At step 104, one or more target sites of the GPCR are identified based on the model parameters learnt from the fitting of the model to the training data set. Since the model parameters express correlation between the surface expression measure and the functional measure, they can be used to investigate which target sites are associated with unusually high or low functional measure values compared to the functional behaviour expected for a given level of surface expression. Hence, the target sites which are most likely to be "druggable" (capable of regulating the level of functional behaviour by being modulated by a drug or other ligand targeting that site) can be predicted, while disambiguating the effects of surface expression on the measurements of functional behaviour. Hence, this can provide better prediction of the sites of a given GPCR that are expected to be good targets for therapeutic treatments.

Figure 7 illustrates steps for generating the training data set used in Figure 6. At step 120, a surface expression assay is performed to determine the surface expression measure for each variant of the set of mutated GPCR variants being tested. The library of mutated GPCR variants used for testing may include variants with single mutations and/or variants with two or more mutations. The library may be selected so that each candidate site of interest has one or more variants of the test population with a mutation at that candidate site (preferably multiple variants should be included with mutations at each candidate site). An example of the surface expression assay is described in more detail below.

At step 122, a functional assay is performed to obtain the functional measure for each variant of the GPCR in the same library of mutated GPCR variants as used for the surface expression assay. For example, the functional assay could be a second surface expression assay performed in the presence of a ligand (the first surface expression assay at step 120 having been performed in absence of the ligand), or could be a signalling assay which probes level of signalling associated with each variant of the GPCR.

At step 124, the training data set is generated comprising a number of pairs of corresponding values of the functional measure and surface expression measure for each variant of the tested library of mutated GPCR variants. The training dataset may also associate each pair of functional/surface expression measure values with information defining the combination of one or more mutations associated with the corresponding GPCR variant. The training data set can be stored for later use, transmitted to another entity on a physical storage medium or via a network, or used immediately for fitting a model to the training data set by the same entity who also performed the surface expression assay and functional assay.

Figure 8 illustrates an example of steps for identifying the one or more target sites of the GPCR at step 104 of Figure 6, based on the model parameters determined from fitting the model to the training set. In general, the one or more target sites are identified based on residuals indicating, for each variant of the GPCR, a difference between the functional measure and a predicted functional measure predicted from the surface expression measure based on the set of model parameters. The residuals express the extent to which the functional measure is higher or lower than what would be expected for the corresponding measured level of surface expression for a given variant. Hence, the residuals associated with a group of variants sharing mutations at a given candidate site of the GPCR can be analysed to make a prediction of whether that candidate site has an influence on functional behaviour for the GPCR for reasons other than merely regulating the level of surface expression. For example, Figure 4 parts a and b illustrates graphically the regression fit to the training data for two examples where the functional measure comprises signalling measures relating to different pathways. The residual for a given variant of the GPCR refers to the distance in the y axis between the corresponding data point and the regression line fitted to the training data in the model. Hence, the variants with greatest magnitude residuals are those whose data points lie furthest from the regression line in the y direction.

For each candidate site, an aggregate measure may be calculated based on the group of residuals associated with mutated variants having a mutation at that site, and the one or more target sites can be selected by ranking the target sites based on their aggregate measure or by selecting sites associated with aggregate measures that meet certain selection criteria. In this way, the target sites most likely to have an influence over functional behaviour of the GPCR can be identified, which can be useful for identifying possible sites that could be targets for drugs or other therapeutic treatments aimed at modulating GPCR function.

The aggregate measure associated with a given candidate site may be derived from the corresponding residuals in different ways. For example, the aggregate measure for a given candidate site may comprise a mean, median, minimum or maximum of the residuals associated with variants having a mutation at the given candidate site. Also, in some examples, a score value for a given candidate site may be derived by applying a statistical test to a group of residuals corresponding to variants having a mutation at that candidate site. The statistical test may be any test that generates a score quantifying the extent to which the residuals in one group corresponding to one candidate site are significantly different to the residuals in another group corresponding to a different candidate site, so that the score from the statistical test may be used to classify candidate sites into different classes. For example, the classes could include activating sites with residuals indicating increased functional measure compared to the predicted functional measure predicted by the model based on the corresponding surface expression measure; inactivating sites with residuals generally indicating decreased functional measures compared to the predicted functional measure, and/or neutral sites which do not appear to either increase or decrease the functional measure compared to the predicted functional measure). For example, the statistical test may be the Mann-Whitney U test, Z-test, or any other statistical test for quantifying differences in distribution of values in one group relative to another group. In the specific examples discussed further below, the test applied was the Mann-Whitney U test followed by FDR correction with the Benjamini / Hochberg method. Hence, by applying a statistical function, a score value may be derived which is indicative of whether the group of residuals for a given candidate site indicates that it is statistically likely that the candidate site has an effect on the target property of interest.

Hence, Figure 8 illustrates an example method for identifying the target sites at step 104 of Figure 8. At step 140, for a given variant of the GPCR selected from the training data set, a predicted functional measure is determined from the surface expression measure for that variant by applying the prediction model defined by the model parameters obtained by the fitting step at step 102 of Figure 6. For example, the prediction model defines a transformation function for mapping the surface expression measure to the predicted functional measure, with the transformation function defined by the model parameters learnt by fitting. At step 142, a residual is determined indicating the difference between the actual functional measure obtained from the training data set for the given variant of the GPCR and the predicted functional measure determined at step 140 for that variant. At step 144, it is determined whether residuals have been determined for all variants of the GPCR variant library used to derive the training data set. If not, then another variant is selected as the given variant and the method returns to step 140 to repeat steps 140, 142 for that variant. Although Figure 8 shows the predicted functional measure and residual being determined sequentially for each variant, it is also possible to parallelise the processing of steps 140 and 142 for multiple variants at a time, to speed up determining the residuals for all variants.

Once residuals have been determined for all of the test library of GPCR variants, at step 148, for each candidate site, an aggregate measure or score value is determined based on the residuals that correspond to GPCR variants having a mutation at that candidate site. The aggregate measure or score value can be determined according to any of the aggregate functions or statistical tests described above.

At step 150, the target sites are selected or ranked according to the aggregate measure or score value associated with each candidate site. For example, the selected target sites may be the N highest ranking sites, where N is a predetermined number of target sites considered an appropriate number of sites suitable for further investigation. Alternatively, selection criteria may be defined, and the selected target sites may be any of the candidate sites whose aggregate measures or score values meet those criteria (e.g. candidate sites having an aggregate measure or score value that exceeds a given threshold may be selected). In some examples, the selection of target sites may depend not only on the aggregate/scoring values derived from the residuals from the model, but also on other considerations, such as the extent to which candidate sites may be considered "druggable" (e.g. pockets on the extracellular surface of the GPCR may be considered better candidates for drug targets than pockets in the transmembrane region or on the intracellular surface of the GPCR, where drugs would need to be engineered to cross the plasma membrane).

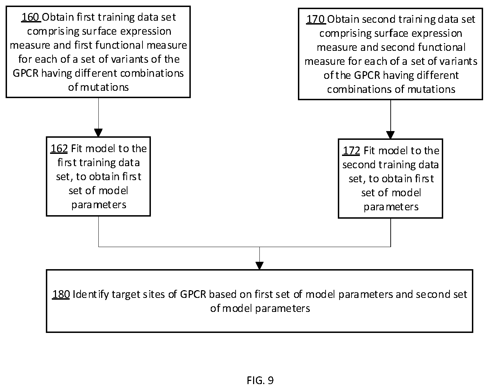

Figure 9 shows an example where two or more different functional measures may be used in combination to identify target sites of the GPCR. The model (e.g. the regression model) may be fit to each of a plurality of training data sets which are based on two or more different functional measures, and the one or more target sites may be identified based on a plurality of sets of model parameters obtained by fitting the model to the plurality of training data sets. This can be helpful as there may be multiple different functional behaviours of interest, and it may be useful to select target sites based on the predicted effect of those target sites on each of those behaviours. For example, the multiple functional behaviours could, for example, including signalling behaviour based on different signalling pathways and/or signalling behaviour in response to different ligands.

At step 160 of Figure 9, a first training data set is obtained specifying, for each of the variants of the GPCR, a surface expression measure and a first functional measure for that variant. The first training data set can be obtained either by performing the steps of Figure 7 for generating the data set, or by obtaining a previously generated data set from data storage circuitry or via a network interface. At step 162, a model is fitted to the first training data set, to obtain a first set of model parameters. The model can be fitted using any of the techniques discussed above with respect to step 102 of Figure 6.

Similarly, at step 170 of Figure 9, a second training data set is obtained specifying, for each of the tested variants of the GPCR, the surface expression measure and a second functional measure for that variant. Again, the second training data set could be generated by the method of Figure 7 or obtained from data storage circuitry or via a network interface. At step 172, the model is fitted to the second training data set to obtain a second set of model parameters (again, based on the techniques discussed above for step 102 of Figure 6).

The first and second functional measures could, for example, be different signalling measures expressing the level of signalling in different scenarios. For example, the first and second functional measures could be based on different signalling pathways (e.g. a G-protein mediated pathway and a β-arrestin mediated pathway), or could be based on signalling responses to different ligands. In some examples, the first functional measure could be a signalling measure and the second functional measure could be a surface expression measure in the presence of a ligand, so as to probe different functional behaviours of interest (not necessarily all signalling related).

At step 180 of Figure 9, the one or more target sites are identified based on the first set of model parameters and the second set of model parameters. For example, this may include performing steps 140 to 148 of Figure 8 separately based on the first set of model parameters and second set of model parameters respectively, to generate for each candidate site a first aggregate measure or score value expressing the contribution to the first functional measure made by that site, and a second aggregate measure or score value expressing the contribution to the second functional measure made by that site. The target sites can then be selected based on prescribed selection criteria made based on the first aggregate measure/score and second aggregate measure/score associated with each site.

The measures based on the first set of model parameters and the second set of model parameters can be combined to select target sites could be done in varying ways, depending on the use case.

In some examples, the one or more target sites comprise one or more target sites for which selection criteria are satisfied based on both the first set of model parameters and the second set of model parameters. This could be useful if the target sites of interest are those which have an effect on both the first functional behaviour expressed by the first functional measure and the second functional behaviour expressed by the second functional measure, and sites which only affect one or other of the first and second functional behaviours may be less of interest.

Alternatively, the one or more target sites could comprise one or more target sites for which selection criteria are satisfied based on either one of the first set of model parameters and the second set of model parameters. In this case, a list of target sites may be derived which are predicted to have an effect on at least one of the first and second functional behaviours quantified by the first and second functional measures.

In some cases, the selection of target sites may be more specific, not only considering whether a site has any influence on functional behaviour of the GPCR, but also whether that influence is an activating or inactivating influence.

For example, in some cases, the selected target sites may be balanced sites predicted to have activating influences on both the first and second functional behaviours, or predicted to have inactivating influence on both the first and second functional behaviour.

In other use cases, it may be of interest to select as the target sites biased sites which have greater influence on one of the first and second functional behaviour than the other (e.g. an activating influence on the first functional behaviour and an inactivating influence on the second functional behaviour, or vice versa). This can be helpful in selecting sites which could be targets for treatment aimed at changing which of the first and second functional behaviours is more active.

Hence, in some cases, the selected one or more target sites may comprise one or more target sites for which selection criteria are satisfied based on one of the first set of model parameters and the second set of model parameters and the selection criteria are not satisfied based on the other of the first set of model parameters and the second set of model parameters.

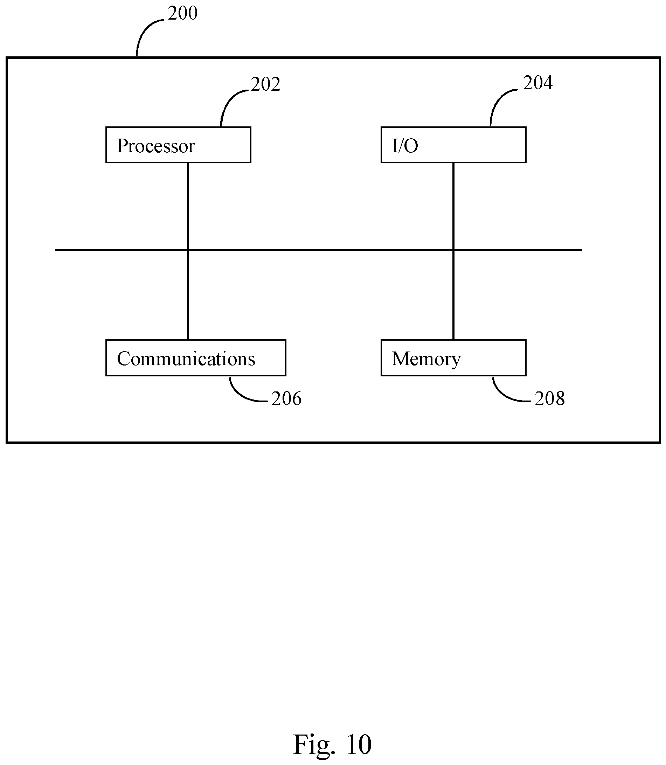

Figure 10 shows a computer apparatus 200 suitable for implementing methods and according to the present disclosure. The apparatus 200 comprises a processor 202, an input-output device 204, a communications unit 206 and computer memory 208. The memory 208 may store program code that, when executed by the processor 202, causes the apparatus 200 to perform any of the computer-implemented methods disclosed herein.

### GPCR signalling assay and tools therefor

Also encompassed by the present invention is a GPCR signalling assay and tools relating to said assay.

DMS can be used to characterize one of surface expression, ligand binding, or signalling of GPCRs in isolation. However, here, the present inventors employ an approach that integrates measurements relating to multiple parameters, which enables an enhanced and more representative understanding of the impact of mutational changes within GPCRs.

In some examples, both signalling and surface expression measurements can be integrated into the methods, which provides an improved means for determining the effects of mutations on GPCRs.

A further improvement afforded by the GPCR signalling assay is that said assay measures a receptor-proximal event.

By receptor-proximal it is meant that the read-out from the assay relies upon direct contact between the activated GPCR and the first intracellular signalling component in a cascade (e.g., a G-protein or variant/functional analogue thereof, β-arrestin or a variant/functional analogue thereof, or a conformation-specific GPCR-binding protein, such as a nanobody). This is in contrast to assays that rely upon the action of molecules further down a signalling cascade, which may even include second messengers (e.g., cAMP).

Utilising an assay that relies upon such receptor-distal activities is susceptible to interference from other cellular signalling pathways, signal amplification, or feedback artefacts, which can disguise or alter the effect of the perturbation being measured (e.g., GPCR mutation).

In one embodiment, there is provided a GPCR signalling assay.

In one embodiment, the GPCR signalling assay comprises a functional measure, wherein the functional measure is a signalling measure.

In one embodiment, the signalling measure is a receptor-proximal measure.

In one embodiment, the signalling measure is a measurement relating to signalling in a:
(i) pathway that is instigated by or involves the activation of a GPCR;
(ii) a G-protein mediated pathway; and/or
(iii) a β-arrestin mediated pathway.

Where multiple signalling pathways, such as two or more, are to be measured at least one of the signalling pathways is a G-protein mediated pathway or a β-arrestin mediated pathway.

In one embodiment, one of the two or more signalling pathways is a G-protein mediated pathway.

In one embodiment, one of the two or more signalling pathways is a β-arrestin mediated pathway.

In one embodiment, the signalling measure is obtained using an assay that does not rely upon adenylyl cyclase.

In one embodiment, the signalling measure is obtained using an assay that does not rely upon endogenous cellular secondary messengers.

In another embodiment, the cellular secondary messenger is cyclic AMP (cAMP).

By rely upon, it will be understood that said molecules are not involved in a signalling cascade upstream of the element of the assay necessary for signal read-out; in other words, removing said molecule would not impact the functionality of the assay, regardless of its involvement downstream of the element necessary for signal read-out.

In one embodiment, the signalling measure is obtained using an assay wherein:
(i) recruitment of a first intracellular signalling component to the GPCR results in the liberation of a transcription activating component; and
(ii) the transcription activating component induces transcription of a reporter gene.

In one embodiment, the first intracellular signalling component is a G-protein.

In one embodiment, the first intracellular signalling component is a variant of a G-protein, such as a miniG protein.

In a further embodiment, first intracellular signalling component is functional analogue of a G-protein that signals via a G-protein dependent pathway.

By functional analogue, it is meant any protein, whether related or unrelated, that is able to bind to the activated state of the GPCR that is recognized by the G-protein.

In one embodiment, the first intracellular signalling component is an antibody; a fragment of an antibody, such as a Fab; an antibody derivative, such as an scFv or nanobody; a modified antibody; or an antibody mimetic.

In one embodiment, the first intracellular signalling component is a nanobody.

In one embodiment, the first intracellular signalling component recognizes an activated conformation of the GPCR.

In one embodiment, the first intracellular signalling component recognizes an activated conformation of the GPCR that biases signalling down a G-protein-dependent pathway.

In one embodiment, the first intracellular signalling component recognizes an activated conformation of the GPCR that biases signalling down a β-arrestin-dependent pathway.

In one embodiment, the first intracellular signalling component recognizes an activated conformation of the GPCR that causes signalling down both G-protein and β-arrestin-dependent pathways.

In one embodiment, liberation of a transcription activating component is achieved by enzymatic cleavage of the transcription activating component from the fusion construct that the uncleaved polypeptide is a part of.

In one embodiment, liberation of a transcription activating component is achieved by enzymatic cleavage of the transcription activating component from GPCR by the first intracellular signalling component.

In one embodiment, liberation of a transcription activating component is achieved by enzymatic cleavage of the transcription activating component from first intracellular signalling component by the GPCR.

In one embodiment, the first intracellular signalling component is a G-protein or variant or functional analogue thereof.

In one embodiment, the first intracellular signalling component is β-arrestin or variant or functional analogue thereof.

In one embodiment, the transcription activating component is a transcription factor or a variant thereof.

In one embodiment, the transcription activating component is Gal4.

In one embodiment, the reporter gene comprises a barcode in the 3' UTR.

In one embodiment, the reporter gene encodes a fluorescent protein.

In one embodiment, the reporter gene is Citrine.

In one embodiment, the reporter gene encodes an enzyme, such as an enzyme capable of producing a catalytic product that is amenable to detection.

In one embodiment,
(i) the first intracellular signalling component is a G-protein or variant thereof that signals via a G-protein dependent pathway;
(ii) the first intracellular signalling component is β-arrestin or variant thereof that signals via a β-arrestin dependent pathway;
(iii) the transcription activating component is a transcription factor of variant thereof, optionally Gal4; and/or
(iv) the reporter gene comprises a barcode that is unique to a specific variant of the GPCR.

The person skilled in the art is aware of suitable reporter genes, which may be selected based on application.

The assay is configured such that the transcription activating component is able to drive transcription of the reporter gene.

In one embodiment, the reporter gene comprises a sequence that is subject to transcriptional regulation by the transcription activating component.

In one embodiment, the reporter gene comprises a Gal4 upstream activator sequence (Gal4 UAS).

It is possible to use multiple pharmacological agents (e.g. ligands) in the signalling assay. In embodiments where multiple ligands are used, two or more different signalling measures may comprise signalling measures depending on signalling in response to different ligands.

In one embodiment, the first and/or second ligand is selected from the group consisting of:
(i) a balanced agonist;
(ii) a biased agonist;
(iii) a balanced antagonist; and
(iv) a biased antagonist.

In one embodiment, the ligand is an endogenous ligand or an exogenous ligand.

In one embodiment, the biased agonist or biased antagonist is an agonist/antagonist of a G-protein mediated pathway or a β-arrestin mediated pathway.

In one embodiment, the balanced agonist or balanced antagonist is an agonist/antagonist of a G-protein mediated pathway and a β-arrestin mediated pathway.

In one embodiment, the GPCR signalling assay comprises providing a cell with any one or more of the nucleic acids or vectors of the invention, namely a nucleic acid molecule or plurality of nucleic acid molecules encoding:
(i) a receptor construct;
(ii) a transducer construct; and/or
(iii) a reporter construct.

In one embodiment, the receptor construct comprises a sequence encoding a variant of a GPCR (e.g., a GPCR comprising one or more mutations as compared to wild-type), wherein said variant is fused to a stimulus sensing component, and a transcription activating component, and wherein said construct comprises a protease cleavage site between said sensing and transcription activating components.

In one embodiment, the transducer construct comprises a sequence encoding a first intracellular signalling component that is fused to a stimulus generating component and a protease component, wherein said protease is able to cleave at the protease cleavage site.

In one embodiment, the reporter construct comprises a sequence encoding a sequence that is subject to transcriptional regulation by the transcription activating component, a reporter gene, and a barcode sequence.

In an assay comprising the above, when stimulated (e.g., by the addition of a ligand) the variant GPCR will signal to the first intracellular signalling domain (e.g., conformational changes in the GPCR result in the recruitment of the first intracellular signalling domain); this results in the transducer construct being brought into proximity of the receptor construct. The stimulus generating component is then induced to produce said stimulus (e.g., by the addition of a chemical substrate, e.g., furimazine where NanoLuciferase is used). The stimulus (e.g., photons), now in proximity to the receptor construct, induces conformational change of the stimulus sensing component (e.g., a LOV domain), which facilitates access of the protease (e.g., a TEV domain) to the now accessible protease cleavage site. The protease cleaves at the cleavage site, liberating the transcription activating component (e.g., Gal4), which is now untethered from the membrane and proceeds to bind to the sequence that is subject to transcriptional regulation by the transcription activating component that is present within the reporter construct. Transcriptional activation by the transcription activating component results in transcription of the reporter gene, which is ultimately measured; the barcode facilitates unambiguous assignation of activity to a specific receptor variant.

In one embodiment:
(i) the heterologous stimulus sensing component is a Light, Oxygen, Voltage sensitive (LOV) domain (LOV);
(ii) the transcription activating component is Gal4;
(iii) the protease cleavage site is a TEV cleavage site;
(iv) the first intracellular signalling component is:
   a. a G-protein or variant thereof;
   b. β-arrestin or a variant thereof; or
   c. a conformation-specific GPCR-binding protein, optionally a nanobody;
(v) the stimulus generating component is a NanoLuciferase;
(vi) the protease component is a TEV protease;
(vii) the sequence that is subject to transcriptional regulation by the transcription activating component is a Gal4 upstream activator sequence (UAS);
(viii) the reporter gene is a fluorescent protein, optionally citrine;
(ix) wherein the sequence that is subject to transcriptional regulation, the reporter gene, and the barcode sequence are operably linked; and/or
(x) the barcode sequence is a barcode sequence that is unique to a specific variant of a GPCR, optionally wherein said barcode sequence is present in the 3'UTR of the reporter construct.

### Tools and reagents

Also encompassed by the present invention are improved tools and reagents that form the basis of the GPCR signalling assays described herein.

In one embodiment, there is provided a nucleic acid molecule or plurality of nucleic acid molecules encoding:
(i) a receptor construct;
(ii) a transducer construct; and/or
(iii) a reporter construct.

By plurality of nucleic acids, it is meant any number of distinct nucleic acid molecule species (i.e., each encoding a different sequence); there may, however, be multiple copies of each species.

In one embodiment, there is provided a nucleic acid molecule or plurality of nucleic acid molecules, wherein:
(i) the receptor construct comprises a sequence encoding a variant of a GPCR, wherein said variant is fused to a stimulus sensing component, and a transcription activating component, and wherein said construct comprises a protease cleavage site between said sensing and transcription activating components;
(ii) the transducer construct comprises a sequence encoding a first intracellular signalling component that is fused to a stimulus generating component and a protease component, wherein said protease is able to cleave at the protease cleavage site; and/or
(iii) the reporter construct comprises a sequence encoding a sequence that is subject to transcriptional regulation by the transcription activating component, a reporter gene, and a barcode sequence.

In one embodiment, there is provided a nucleic acid molecule or plurality of nucleic acid molecules, wherein:
(i) the heterologous stimulus sensing component is a Light, Oxygen, Voltage sensitive (LOV) domain (LOV);
(ii) the transcription activating component is Gal4;
(iii) the protease cleavage site is a TEV cleavage site;
(iv) the first intracellular signalling component is:
   a. a G-protein or variant thereof;
   b. β-arrestin or a variant thereof; or
   c. a conformation-specific GPCR-binding protein, optionally a nanobody;
(v) the stimulus generating component is a NanoLuciferase;
(vi) the protease component is a TEV protease;
(vii) the sequence that is subject to transcriptional regulation by the transcription activating component is a Gal4 upstream activator sequence (UAS);
(viii) the reporter gene is a fluorescent protein, optionally citrine;
(ix) wherein the sequence that is subject to transcriptional regulation, the reporter gene, and the barcode sequence are operably linked; and/or
(x) the barcode sequence is a barcode sequence that is unique to a specific variant of a GPCR, optionally wherein said barcode sequence is present in the 3'UTR of the reporter construct.

In one embodiment, there is provided a nucleic acid molecule or plurality of nucleic acid molecules, wherein:
(i) the receptor construct, the transducer construct, and the reporter construct are encoded in different nucleic acid molecules;
(ii) the receptor construct, the transducer construct, and the reporter construct are encoded in the same nucleic acid molecule;
(iii) two of the receptor construct, the transducer construct, and/or the reporter construct are encoded in the same nucleic acid molecule, and the remaining is encoded in a different nucleic acid molecule.

In one embodiment, the receptor construct and the reporter construct are encoded in the same nucleic acid molecule.

In one embodiment, there is provided a vector or plurality of vectors encoding the nucleic acid molecule or plurality of nucleic acid molecules of the invention.

In one embodiment, there is provided one or more polypeptides encoded by the nucleic acid molecules or vectors of the invention.

In one embodiment, there is provided a cell comprising the nucleic acid molecule or plurality of nucleic acid molecules, or the vector or plurality of vectors of the invention.

In one embodiment, the cell comprises one or more nucleic acid molecules or one or more vectors encoding the receptor construct and the reporter construct.

At any time the cell may comprise any number of the nucleic acid molecules or vectors of the invention. These may be supplemented with any one or more of the remaining nucleic acid molecules or vectors of the invention subsequently.

In one embodiment, the nucleic acid molecules or vectors may be free within the cell or may be integrated into the genome of the cell.

In one embodiment, the cell comprises one or more nucleic acid molecules or vectors according to the invention, wherein said nucleic acid molecules or vectors are recombined into the genome of the cell.

For example, a cell may comprise nucleic acids/vectors comprising the receptor construct and reporter construct. Such a cell may later be supplemented with the transducer construct.

### Cell surface expression assay

Cell surface expression measurements may be integrated into the GPCR signalling assay of the invention.

GPCR signalling assays typically don't measure the amount of GPCR present on the cell surface. In the absence of such a measurement, it cannot be determined whether any apparently reduced activity is indeed due to a less active GPCR or due to the fact that there are fewer GPCRs present at the cell surface.

In one embodiment, the surface expression measure is a measurement obtained using an assay that comprises quantification and/or separation of cells expressing the GPCR variant, wherein the GPCR variant is labelled.

In one embodiment, the GPCR is fluorescently labelled.

In one embodiment, the fluorescent labelling is provided using a conjugated antibody or variant thereof.

In one embodiment, the quantification and/or separation is achieved using fluorescence-activated cell sorting (FACS).

### Pharmacological Rescue

The cell surface expression assay can further be used to determine the level of pharmacological rescue by a ligand for the GPCR (e.g. where use of a ligand is used to traffic the mutated GPCR to the plasma membrane of the cell). It can be incorporated into the method for identifying one or more target sites of a GPCR.

In order to do so, the surface expression of a variant is determined in the absence of a ligand for the GPCR. A second measurement of the surface expression of the variant is made in the presence of a ligand for the GPCR,

In one embodiment for a given variant of the GPCR:
the surface expression measure is a first surface expression measure indicative of surface expression of the given variant of the GPCR in absence of a ligand; and
the functional measure is a second surface expression measure indicative of surface expression of the given variant of the GPCR in presence of a ligand.

In one embodiment, the ligand is:
(i) a balanced agonist;
(ii) a biased agonist;
(iii) a balanced antagonist; or
(iv) a biased antagonist.

In one embodiment, the ligand increases the expression of the variant of the GPCR at the cell surface.

In one embodiment, the ligand decreases the expression of the variant of the GPCR at the cell surface.

In one embodiment, the ligand is a known ligand of the GPCR.

In one embodiment, the ligand has an unknown binding site on the GPCR.

Comparison of all variant surface expression scores in control conditions versus the ligand rescue condition can then be made. A loess curve can be fit to the data, which allows identification of variants whose surface expression is rescued by the ligand.

A residual is then calculated for all variants as the distance between the loess curve and the surface expression in the ligand rescue condition. Then, for each position, a statistical test (for example: Mann-Whitney U test, t-test, Z-test, etc.) compares the residuals of that position with the residuals of variants at all other positions.

Alternatively, the magnitude (for example, mean, median, maximum, etc.) of residuals at each position is compared.

This method can be used to map the binding site of molecules with an unknown binding site.

### β₂AR

The β₂-adrenergic receptor (β₂AR), is a G protein coupled receptor that binds adrenaline.. It is a G_{S} coupled receptor, meaning that activation of the receptor results in an increase of cAMP, ultimately resulting in physiological responses such as the relaxation of smooth muscle and bronchodilation β₂AR is a well studied GPCR and served as the basis for pioneering work on the structure of GPCRs, which have since provided insight into other GPCRs.

Wild-type human β₂AR comprises the amino acid sequence set forth in SEQ ID NO: 1 herein.

MGQPGNGSAFLLAPNRSHAPDHDVTQQRDEVWVVGMGIVMSLIVLAIVFGNVLVITAIAKFE
RLQTVTNYFITSLACADLVMGLAVVPFGAAHILMKMWTFGNFWCEFWTSIDVLCVTASIETL
CVIAVDRYFAITSPFKYQSLLTKNKARVIILMVWIVSGLTSFLPIQMHWYRATHQEAINCYANE
TCCDFFTNQAYAIASSIVSFYVPLVIMVFVYSRVFQEAKRQLQKIDKSEGRFHVQNLSQVEQ
DGRTGHGLRRSSKFCLKEHKALKTLGIIMGTFTLCWLPFFIVNIVHVIQDNLIRKEVYILLNWI
GYVNSGFNPLIYCRSPDFRIAFQELLCLRRSSLKAYGNGYSSNGNTGEQSGYHVEQEKENK
LLCEDLPGTEDFVGHQGTVPSDNIDSQGRNCSTNDSLL

It will be understood that all references to β₂AR sequence positions herein refer to the wild-type β₂AR amino acid sequence as set out in SEQ ID NO: 1.

### Wild-type β₂AR amino acid sequence with sequence positions

M1, G2, Q3, P4, G5, N6, G7, S8, A9, F10, L11, L12, A13, P14, N15, R16, S17, H18, A19, P20, D21, H22, D23, V24, T25, Q26, Q27, R28, D29, E30, V31, W32, V33, V34, G35, M36, G37, I38, V39, M40, S41, L42, I43, V44, L45, A46, I47, V48, F49, G50, N51, V52, L53, V54, I55, T56, A57, I58, A59, K60, F61, E62, R63, L64, Q65, T66, V67, T68, N69, Y70, F71, I72, T73, S74, L75, A76, C77, A78, D79, L80, V81, M82, G83, L84, A85, V86, V87, P88, F89, G90, A91, A92, H93, I94, L95, M96, K97, M98, W99, T100, F101, G102, N103, F104, W105, C106, E107, F108, W109, T110, S111, I112, D113, V114, L115, C116, V117, T118, A119, S120, 1121, E122, T123, L124, C125, V126, I127, A128, V129, D130, R131, Y132, F133, A134, I135, T136, S137, P138, F139, K140, Y141, Q142, S143, L144, L145, T146, K147, N148, K149, A150, R151, V152, I153, I154, L155, M156, V157, W158, I159, V160, S161, G162, L163, T164, S165, F166, L167, P168, I169, Q170, M171, H172, W173, Y174, R175, A176, T177, H178, Q179, E180, A181, 1182, N183, C184, Y185, A186, N187, E188, T189, C190, C191, D192, F193, F194, T195, N196, Q197, A198, Y199, A200, 1201, A202, S203, S204, I205, V206, S207, F208, Y209, V210, P211, L212, V213, I214, M215, V216, F217, V218, Y219, S220, R221, V222, F223, Q224, E225, A226, K227, R228, Q229, L230, Q231, K232, I233, D234, K235, S236, E237, G238, R239, F240, H241, V242, Q243, N244, L245, S246, Q247, V248, E249, Q250, D251, G252, R253, T254, G255, H256, G257, L258, R259, R260, S261, S262, K263, F264, C265, L266, K267, E268, H269, K270, A271, L272, K273, T274, L275, G276, I277, I278, M279, G280, T281, F282, T283, L284, C285, W286, L287, P288, F289, F290, I291, V292, N293, I294, V295, H296, V297, I298, Q299, D300, N301, L302, I303, R304, K305, E306, V307, Y308, I309, L310, L311, N312, W313, I314, G315, Y316, V317, N318, S319, G320, F321, N322, P323, L324, I325, Y326, C327, R328, S329, P330, D331, F332, R333, I334, A335, F336, Q337, E338, L339, L340, C341, L342, R343, R344, S345, S346, L347, K348, A349, Y350, G351, N352, G353, Y354, S355, S356, N357, G358, N359, T360, G361, E362, Q363, S364, G365, Y366, H367, V368, E369, Q370, E371, K372, E373, N374, K375, L376, L377, C378, E379, D380, L381, P382, G383, T384, E385, D386, F387, V388, G389, H390, Q391, G392, T393, V394, P395, S396, D397, N398, I399, D400, S401, Q402, G403, R404, N405, C406, S407, T408, N409, D410, S411, L412, L413

Any and all references to positions within β₂AR are intended to encompass the equivalent positions/residues in an analogous sequence, even if said sequence is not identical to SEQ ID NO: 1. For example, where a sequence is a truncated or elongated sequence relative to SEQ ID NO: 1, P4 will refer to the P that is present in the equivalent position regardless of if it is residue 4 when counted from the N-terminus.

The person skilled in the art is able to determine equivalent positions in analogous sequences, e.g., using widely available alignment tools such as BLAST or EMBOSS, or manually.

With respect to the method of modulating the activity of β₂AR, the β₂AR polypeptides, the β₂AR binding molecules, or any other aspect of the present invention, the activity referred to is any signalling activity that arises from β₂AR.

In one embodiment, the activity is G-protein dependent signalling activity.

In one embodiment, the activity is β-arrestin dependent signalling activity.

In one embodiment, the activity is signalling activity down one signalling pathway.

Signalling activity may be modulated in respect of one signalling pathway independently of any other (e.g., in response to a biased agonist), or in respect of any number of a plurality of signalling pathways simultaneously (e.g., in response to a balanced agonist).

In one embodiment, the activity is signalling activity down more than one signalling pathway.

In one embodiment, the activity is signalling activity down two signalling pathway.

In one embodiment, the activity is β-arrestin dependent signalling activity and G-protein dependent signalling activity.

The activity may be the activity that arises in the presence of any given ligand or combination of ligands of β₂AR.

### Modulating the activity of β₂AR

In accordance with the inventors discovery of allosteric sites within β₂AR, provided herein is a method of modulating the activity of β₂AR, the method comprising the step of mutating one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

By "modulating the activity of β₂AR" it is meant the alteration of any signalling activity arising directly from a variant of β₂AR as compared with the same activity (e.g., same signalling pathway) of an unmodified, wild-type β₂AR. It will be understood that encompassed within the present invention are variants of the wild-type and variant β₂AR proteins that include, for example, tags for enabling purification or identification. The person skilled in the art would understand that a comparison of activity could be carried out using equivalent constructs that differ in the amino acids present within the β₂AR sequence region defined by SEQ ID NO: 1. For example, a β₂AR comprising SEQ ID NO: 1 in addition to a protein tag (e.g., a GFP tag) may be compared with a variant β₂AR comprising a modified version of SEQ ID NO: 1 in addition to the same tag.

In one embodiment, one, two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, thirty one, thirty two, thirty three, thirty four, thirty five, thirty six, thirty seven, thirty eight, thirty nine, forty, forty one, forty two, forty three, forty four, forty five, forty six, forty seven, forty eight, forty nine, fifty, fifty one, fifty two, fifty three, fifty four, fifty five, fifty six, fifty seven, fifty eight, fifty nine, sixty, sixty one, sixty two, sixty three, sixty four, sixty five, sixty six, sixty seven, sixty eight, sixty nine, seventy, seventy one, seventy two, seventy three, seventy four, seventy five, seventy six, seventy seven, seventy eight, seventy nine, eighty, eighty one, eighty two, eighty three, eighty four, eighty five, eighty six, eighty seven, eighty eight, eighty nine, ninety, ninety one, ninety two, ninety three, ninety four, ninety five, ninety six, ninety seven, ninety eight, ninety nine, one hundred, one hundred and one, one hundred and two, one hundred and three, one hundred and four, one hundred and five, one hundred and six, one hundred and seven, one hundred and eight, one hundred and nine, one hundred and ten, one hundred and eleven, one hundred and twelve, one hundred and thirteen, one hundred and fourteen, one hundred and fifteen, one hundred and sixteen, one hundred and seventeen, one hundred and eighteen, one hundred and nineteen, one hundred and twenty, one hundred and twenty one, one hundred and twenty two, one hundred and twenty three, one hundred and twenty four, one hundred and twenty five, one hundred and twenty six, one hundred and twenty seven, one hundred and twenty eight, one hundred and twenty nine, one hundred and thirty, one hundred and thirty one, one hundred and thirty two, one hundred and thirty three, one hundred and thirty four, or one hundred and thirty five residues are mutated

In one embodiment, the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.

In one embodiment, the one or more residues is selected from the group consisting of: V67, A76, and L145.

In one embodiment, the one or more residues is selected from the group consisting of: V54, F71, L75, A78, L80, W99, S111, V126, I153, V160, F166, A181, A200, P211, Y308, Y316, and P330.

In one embodiment, the one or more residues is selected from the group consisting of: V54, V67, F71, L75, A76, A78, L80, W99, S111, V126, L145, I153, V160, F166, A181, A200, P211, Y308, Y316, and P330.

In one embodiment, the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, I182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407.

In one embodiment, the one or more residues is selected from the group consisting of: S17, P20, M36, I43, L64, A85, A91, M96, G102, W105, P168, Y174, Y185, D234, H269, C285, V295, and N352.

In one embodiment, the one or more residues is selected from the group consisting of: N6, P14, V24, R28, V44, F49, G50, I94, D130, I182, E225, Q229, F240, S246, G255, I277, I314, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, L64, A85, A91, I94, M96, G102, W105, D130, P168, Y174, I182, Y185, E225, Q229, D234, F240, S246, G255, H269, I277, C285, V295, 1314, S346, G351, N352, S355, N357, T360, E362, V368, E369, K372, E373, N374, G383, D386, V388, Q402, and S407.

In one embodiment, the one or more residues is selected from the group consisting of: S17, P20, M36, I43, L64, V67, A76, A85, A91, M96, G102, W105, L145, P168, Y174, Y185, D234, H269, C285, V295, and N352.

In one embodiment, the one or more residues is selected from the group consisting of: N6, P14, V24, R28, V44, F49, G50, V54, F71, L75, A78, L80, I94, W99, S111, V126, D130, I153, V160, F166, A181, 1182, A200, P211, E225, Q229, F240, S246, G255, I277, Y308, I314, Y316, P330, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, V54, L64, V67, F71, L75, A76, A78, L80, A85, A91, I94, M96, W99, G102, W105, S111, V126, D130, L145, I153, V160, F166, P168, Y174, A181, I182, Y185, A200, P211, E225, Q229, D234, F240, S246, G255, H269, I277, C285, V295, Y308, I314, Y316, P330, S346, G351, N352, S355, N357, T360, E362, V368, E369, K372, E373, N374, G383, D386, V388, Q402, and S407.

In one embodiment, the one or more residues is selected from the group consisting of: N15, Q26, G37, D79, M82, V87, L95, F101, C106, D113, V114, V117, T118, A119, I121, L124, 1127, A128, V129, R131, P138, F139, Y141, S161, T164, S165, C184, N187, C190, C191F193, Y199, S203, S204, S207, F208, Y209, V218, Y219, S236, E268, L272, G276, I278, G280, T283, W286, F289, F290, N293, I294, 312, W313, G315, N318, N322, P323, I325, Y326, C327, R328, S329, K375, and N398.

In one embodiment, the one or more residues is selected from the group consisting of: D79, M82, C106, D113, V114, V117, T118, A119, I121, I127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.

In one embodiment, the one or more residues is selected from the group consisting of: N15, Q26, G37, V87, L95, F101, L124, N187, V218, S236, E268, L272, G276, T283, W313, P323, K375, and N398.

In one embodiment, the one or more residues is located within an allosteric site.

In one embodiment, the modulating is activating or inactivating.

In one embodiment, the modulating is activating.

In one embodiment, the modulating is inactivating.

Any suitable assay may be used to reliably compare the activity of β₂AR.

Notwithstanding, the general availability of assays to the skilled person, the inventors provide herein improved assays that facilitate measurement of GPCR activity using a receptor-proximal read out.

In another embodiment, the method of modulating the activity of β₂AR comprises mutating one residue.

The present inventors have generated an array of single point mutants of β₂AR, which enabled determination of the allosteric effects of said residue/site.

Herein, the term "site" is taken to mean a region of β₂AR; the site may comprise or consist of a single amino acid residue or more than one residue (e.g., a group of residues). Where the site comprises more than one amino acid, the amino acids may be continuous or discontinuous in the primary sequence or on the surface of the folded protein (i.e., comprising a continuous or discontinuous patch or site).

In one embodiment, the method is an *in vitro* method.

In one embodiment, the method is an *in vivo* method.

In one embodiment, the method is an *ex vivo* method.

In one embodiment, the method is performed *ex vivo.*

It will be understood that mutating one or more residues may refer to mutational changes made directly at the amino acid level or mutational changes made at a nucleic acid level, e.g., by altering the nature of the codon encoding the corresponding β₂AR residue.

Said mutating may result in a stable, heritable change, e.g., as a result of mutation of genomic DNA, such as in a cell; or it may result in a transient change, e.g., as a result of the introduction of mRNA encoding a β₂AR comprising said mutated residues.

In one embodiment, the mutating is carried out by means of mutating the nucleic acid sequence encoding the one or more residues.

Mutating the one or more residues according to the invention may be carried out by any suitable means.

Said mutation may be carried out, at the nucleic acid level, by PCR-based techniques, TALEN based gene editing; CRISPR/CAS based gene editing, etc.

In one embodiment, the mutating is carried out using CRISPR/CAS based gene editing technologies, or variants thereof.

In one embodiment, there is provided a β₂AR produced according to the method of the invention.

Herein, where selections from a group may be made it is to be understood that, unless otherwise stated, said selections are independent of one another.

### β₂AR variants

β₂AR may be modified or mutated to produce variants of β₂AR, as compared to a wild-type β₂AR. Said variants may have modified activities relative to the wild-type β₂AR.

In one embodiment, the activity of the polypeptide is modulated relative to the activity of wild-type β₂AR.

In one embodiment, the modulation is an increase or a decrease in activity relative to the activity of wild-type β₂AR.

In one embodiment, the modulation is an increase in activity relative to the activity of wild-type β₂AR.

Any of the residues identified herein may be mutated to constitute a variant comprising any other naturally or non-naturally occurring amino acids.

In one embodiment, the variant comprises a mutation that constitutes a conservative mutation.

In one embodiment, the variant comprises a mutation that constitutes a non-conservative mutation.

In one embodiment the polypeptide encoding a β₂AR variant comprises a mutation, relative to a wild-type β₂AR, that constitutes a mutation of any one or more of the identified residues to any one or more of the amino acids selected from the group consisting of: A, R, N, D, C, E, Q, G, H, I, L, K, M, F, P, S, T, W, Y, and V.

In accordance with the invention herein providing a method of modulating the activity of β₂AR comprising mutating one or more residues, there is also provided herein polypeptides encoding a β₂AR variant produced according to the method of the invention.

A variant may also be referred to as a modified or mutated β₂AR, in which each will be understood to be a variant or modification of, or mutated relative to a wild-type β₂AR, e.g., comprising or consisting of SEQ ID NO: 1.

### β2AR binding molecules

In accordance with the discovery of sites that allosterically regulate β₂AR activity, the present invention also provides binding molecules that target said allosteric sites. Said molecules may modulate the activity of β₂AR, e.g., wild-type β₂AR.

Provided herein, a binding molecule which binds to one or more target sites on a β2-adrenergic receptor (β₂AR), wherein the one or more target sites comprises one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

In one embodiment, the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, I182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407.

In one embodiment, the one or more residues is selected from the group consisting of: N15, Q26, G37, V87, L95, F101, L124, N187, V218, S236, E268, L272, G276, T283, W313, P323, K375, and N398.

In one embodiment, the one or more residues is selected from the group consisting of: S17, P20, M36, I43, L64, A85, A91, M96, G102, W105, P168, Y174, Y185, D234, H269, C285, V295, and N352.

In one embodiment, the one or more residues is selected from the group consisting of: N6, P14, V24, R28, V44, F49, G50, I94, D130, I182, E225, Q229, F240, S246, G255, I277, I314, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, L64, A85, A91, I94, M96, G102, W105, D130, P168, Y174, I182, Y185, E225, Q229, D234, F240, S246, G255, H269, I277, C285, V295, 1314, S346, G351, N352, S355, N357, T360, E362, V368, E369, K372, E373, N374, G383, D386, V388, Q402, and S407.

In one embodiment, the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.

In one embodiment, the one or more residues is selected from the group consisting of: D79, M82, C106, D113, V114, V117, T118, A119, I121, I127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.

In one embodiment, the one or more residues is selected from the group consisting of: V67, A76, and L145.

In one embodiment, the one or more residues is selected from the group consisting of: V54, F71, L75, A78, L80, W99, S111, V126, I153, V160, F166, A181, A200, P211, Y308, Y316, and P330

In one embodiment, the one or more residues is selected from the group consisting of: V54, V67, F71, L75, A76, A78, L80, W99, S111, V126, L145, I153, V160, F166, A181, A200, P211, Y308, Y316, and P330.

In one embodiment, the one or more residues is selected from the group consisting of: S17, P20, M36, I43, L64, V67, A76, A85, A91, M96, G102, W105, L145, P168, Y174, Y185, D234, H269, C285, V295, and N352.

In one embodiment, the one or more residues is selected from the group consisting of: N6, P14, V24, R28, V44, F49, G50, V54, F71, L75, A78, L80, I94, W99, S111, V126, D130, I153, V160, F166, A181, 1182, A200, P211, E225, Q229, F240, S246, G255, I277, Y308, I314, Y316, P330, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, V54, L64, V67, F71, L75, A76, A78, L80, A85, A91, I94, M96, W99, G102, W105, S111, V126, D130, L145, I153, V160, F166, P168, Y174, A181, I182, Y185, A200, P211, E225, Q229, D234, F240, S246, G255, H269, I277, C285, V295, Y308, I314, Y316, P330, S346, G351, N352, S355, N357, T360, E362, V368, E369, K372, E373, N374, G383, D386, V388, Q402, and S407.

In one embodiment, the one or more residues is selected from the group consisting of: N15, Q26, G37, D79, M82, V87, L95, F101, C106, D113, V114, V117, T118, A119, I121, L124, 1127, A128, V129, R131, P138, F139, Y141, S161, T164, S165, C184, N187, C190, C191, F193, Y199, S203, S204, S207, F208, Y209, V218, Y219, S236, E268, L272, G276, I278, G280, T283, W286, F289, F290, N293, I294, N312, W313, G315, N318, N322, P323, I325, Y326, C327, R328, S329, K375, and N398.

In one embodiment, the one or more target sites is one or more allosteric sites.

In one embodiment, the one or more target sites is an allosteric site.

In one embodiment, one or more of the one or more residues is surface exposed or solvent accessible in at least one conformational state of the β₂AR.

In one embodiment, the one or more target sites is surface exposed.

In one embodiment, the one or more target sites is solvent accessible.

In one embodiment, at least one of the one or more target sites is solvent accessible.

In one embodiment, the one or more target sites is partially solvent accessible.

In one embodiment, the one or more target sites form a pocket or patch:
(i) on the intracellular or extracellular surfaces of β₂AR;
(ii) at or near the transmembrane domain of β₂AR;
(iii) within the central core of the helical bundle of β₂AR.

In one embodiment, the one or more target sites is accessible for binding by one or more binding molecules.

In one embodiment, the one or more target sites is accessible for binding by one or more binding molecules by a lock and key, or an induced fit mechanism.

In one embodiment, binding by one or more binding molecules requires binding of one or more further binding molecules (e.g., ligands) of β₂AR.

The target sites herein may be targeted by a binding molecule. Said binding molecules may have therapeutic benefit.

In one embodiment, the one or more target sites is druggable.

In one embodiment, the binding molecule is:
(i) a polypeptide;
(ii) a nucleic acid; or
(iii) a small molecule.

In one embodiment, the binding molecule is a small molecule or a biologic.

In one embodiment, the binding molecule is a nucleic acid.

In one embodiment, the binding molecule is a polypeptide.

Any reference to a polypeptide or protein herein is to be understood as interchangeable.

In one embodiment, the binding molecule is an antibody, or a mimetic or derivative thereof, optionally an affibody, a nanobody, a Fab fragment, or a scFv.

In one embodiment, the binding molecule is an antibody or a derivative thereof, optionally a nanobody, a Fab fragment, a scFv, or the like.

In one embodiment, the binding molecule is an antibody mimetic, such as an affibody.

In another embodiment, the binding molecule is a DARPIN.

In one embodiment, the binding molecule is an aptamer.

In one embodiment, the binding molecule is:
(i) a DNA
(ii) an RNA
(iii) a DNA/RNA hybrid
(iv) a modified DNA; or
(v) a modified RNA.

By modified DNA or RNA it is meant a DNA or RNA comprising non-naturally occurring modifications (e.g., chemical groups, such as phosphorothioate internucleoside linkages) as compared with DNA and RNA found *in vivo.*

In one embodiment, the binding molecule is a small molecule. In another embodiment, the binding molecule is a drug-like small molecule.

In one embodiment, the binding molecule modulates the activity of β₂AR.

In one embodiment, the modulating is activating or inactivating.

In one embodiment, the binding molecule modulates the activity of β₂AR, optionally wherein the modulating is an increase or a decrease in activity.

In one embodiment, the binding molecule increases the activity of β₂AR relative to the activity of β₂AR in the absence of the binding molecule.

In one embodiment, the binding molecule decreases the activity of β₂AR relative to the activity of β₂AR in the absence of the binding molecule.

In one embodiment, the modulating is activating.

In one embodiment, the activity of β₂AR is the activity on:
(i) a G-protein; and/or
(ii) β-arrestin.

In one embodiment, the activity that is modulated is G-protein dependent signalling activity.

In one embodiment, the activity that is modulated is β-arrestin dependent signalling activity.

In one embodiment, the activity that is modulated is signalling activity down one signalling pathway.

Signalling activity may be modulated in respect of one signalling pathway independently of any other (e.g., in response to a biased agonist), or in respect of any number of a plurality of signalling pathways simultaneously (e.g., in response to a balanced agonist).

In one embodiment, the activity that is modulated is signalling activity down more than one signalling pathway.

In one embodiment, the activity that is modulated is signalling activity down two signalling pathway.

In one embodiment, the activity that is modulated is β-arrestin dependent signalling activity and G-protein dependent signalling activity.

The activity that is modulated may be the activity of any given ligand or combination of ligands of β₂AR.

### Polypeptides

In one aspect, provided herein is a polypeptide encoding a β₂-adrenergic receptor (β₂AR) variant, wherein the polypeptide comprises a mutation, relative to a wild-type β₂AR, at one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

In one embodiment, the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, I182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407.

In one embodiment, the one or more residues is selected from the group consisting of: N15, Q26, G37, V87, L95, F101, L124, N187, V218, S236, E268, L272, G276, T283, W313, P323, K375, and N398.

In one embodiment, the one or more residues is selected from the group consisting of: S17, P20, M36, I43, L64, A85, A91, M96, G102, W105, P168, Y174, Y185, D234, H269, C285, V295, and N352.

In one embodiment, the one or more residues is selected from the group consisting of: N6, P14, V24, R28, V44, F49, G50, I94, D130, I182, E225, Q229, F240, S246, G255, I277, I314, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, L64, A85, A91, I94, M96, G102, W105, D130, P168, Y174, I182, Y185, E225, Q229, D234, F240, S246, G255, H269, I277, C285, V295, 1314, S346, G351, N352, S355, N357, T360, E362, V368, E369, K372, E373, N374, G383, D386, V388, Q402, and S407.

In one embodiment, the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.

In one embodiment, the one or more residues is selected from the group consisting of: D79, M82, C106, D113, V114, V117, T118, A119, I121, I127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.

In one embodiment, the one or more residues is selected from the group consisting of: V67, A76, and L145.

In one embodiment, the one or more residues is selected from the group consisting of: V54, F71, L75, A78, L80, W99, S111, V126, I153, V160, F166, A181, A200, P211, Y308, Y316, and P330.

In one embodiment, the one or more residues is selected from the group consisting of: V54, V67, F71, L75, A76, A78, L80, W99, S111, V126, L145, I153, V160, F166, A181, A200, P211, Y308, Y316, and P330.

In one embodiment, the one or more residues is selected from the group consisting of: S17, P20, M36, I43, L64, V67, A76, A85, A91, M96, G102, W105, L145, P168, Y174, Y185, D234, H269, C285, V295, and N352.

In one embodiment, the one or more residues is selected from the group consisting of: N6, P14, V24, R28, V44, F49, G50, V54, F71, L75, A78, L80, I94, W99, S111, V126, D130, I153, V160, F166, A181, 1182, A200, P211, E225, Q229, F240, S246, G255, I277, Y308, I314, Y316, P330, S346, G351, S355, N357, T360, E362, V368, E369, K372, E373, N374, G383, D386, V388, Q402, and S407.

In one embodiment, the one or more residues is selected from the group consisting of: N6, P14, S17, P20, V24, R28, M36, I43, V44, F49, G50, V54, L64, V67, F71, L75, A76, A78, L80, A85, A91, I94, M96, W99, G102, W105, S111, V126, D130, L145, I153, V160, F166, P168, Y174, A181, I182, Y185, A200, P211, E225, Q229, D234, F240, S246, G255, H269, I277, C285, V295, Y308, I314, Y316, P330, S346, G351, N352, S355, N357, T360, E362, V368, E369, K372, E373, N374, G383, D386, V388, Q402, and S407.

In one embodiment, the one or more residues is selected from the group consisting of: N15, Q26, G37, D79, M82, V87, L95, F101, C106, D113, V114, V117, T118, A119, I121, L124, 1127, A128, V129, R131, P138, F139, Y141, S161, T164, S165, C184, N187, C190, C191, F193, Y199, S203, S204, S207, F208, Y209, V218, Y219, S236, E268, L272, G276, I278, G280, T283, W286, F289, F290, N293, I294, N312, W313, G315, N318, N322, P323, I325, Y326, C327, R328, S329, K375, and N398.

In one embodiment, the one or more residues is located within an allosteric site.

In one embodiment, the activity of the polypeptide is modulated relative to the activity of a wild-type β₂AR, optionally wherein the modulated activity is an increase or a decrease in activity.

In one embodiment, the activity is the activity on:
(i) a G-protein; and/or
(ii) β-arrestin.

### Nucleic acids

In one aspect, provided herein is a nucleic acid encoding:
(i) the binding molecule of the invention; and/or
(ii) the polypeptide of the invention.

In one embodiment, the nucleic acid encodes a polypeptide according to the invention.

In one embodiment, the nucleic acid encodes a binding molecule according to the invention, optionally wherein said binding molecule is a polypeptide or a nucleic acid.

It will be understood that, where the β₂AR binding molecule of the invention is a polypeptide or a nucleic acid based binding molecule (e.g., DNA or RNA), said binding molecule may be encoded by a nucleic acid molecule according to the foregoing.

In one embodiment, the nucleic acid is RNA or DNA, optionally wherein the nucleic acid is modified, unmodified, naturally occurring or synthetic.

In one embodiment, the nucleic acid is RNA.

In one embodiment, the nucleic acid is DNA.

In one embodiment, the nucleic acid is:
(i) a DNA
(ii) an RNA
(iii) a DNA/RNA hybrid
(iv) a modified DNA; or
(v) a modified RNA.

By modified DNA or RNA it is meant a DNA or RNA comprising non-naturally occurring modifications (e.g., chemical groups, such as phosphorothioate internucleoside linkages) as compared with DNA and RNA found *in vivo.*

It will be understood that both RNA and DNA can be considered to encode RNAs, DNAs, and polypeptides. For example a DNA can be amplified to produce further DNA, or transcribed to produce an RNA, optionally wherein said RNA is then translated to produce a polypeptide; an RNA can be reverse transcribed to form a DNA, translated into protein, or amplified into further RNAs.

In one embodiment, the nucleic acid is modified, unmodified, naturally occurring or synthetic.

In another aspect, there is provided an expression cassette comprising the nucleic acid of the invention.

In a further aspect, there is provided a vector comprising the nucleic acid or the expression cassette of the invention.

Expression cassettes and/or vectors that enable the transcription and/or translation of nucleotide sequences of interest are known in the art and may be selected by the person skilled in the art dependent upon application. For example, cell-type specific promotors may be chosen to restrict expression of a payload to certain cell types.

### Cells

In one aspect, there is provided a cell comprising the binding molecule, the polypeptide, the nucleic acid, the expression cassette, or the vector of the invention.

In one embodiment, there is provided a cell comprising the binding molecule of the invention and/or a nucleic acid encoding the binding molecule of the invention.

In one embodiment, there is provided a cell comprising the polypeptide of the invention, and/or a nucleic acid encoding the polypeptide of the invention.

In one embodiment, there is provided a cell comprising the nucleic acid of the invention.

In one embodiment, there is provided a cell comprising the expression cassette of the invention.

In one embodiment, there is provided a cell comprising the vector of the invention.

Given that the presence of the aforementioned features in a cell is not mutually exclusive, also provided is a cell comprising any combination of the binding molecule, the polypeptide, the nucleic acid, the expression cassette, and/or the vector the invention.

In one embodiment, there is provided cell comprising the nucleic acid, the expression cassette, or the vector of the invention.

In one embodiment, the cell is a prokaryotic cell, optionally a bacterial cell.

In one embodiment, the cell is a eukaryotic cell.

In one embodiment, the cell is a yeast cell.

In some embodiments, the cell is a mammalian cell, preferably a human cell.

In one embodiment, the cell is an *in vitro* cell.

The cell may be a cell derived from a human, optionally a human suffering from a disease or disorder, or susceptible to a disease or disorder, or a human with no known disease or susceptibility thereto.

In one embodiment, the cell is a human cell derived from a subject suffering from or susceptible to a disease. In some embodiments, said disease is a disease associated with β₂AR.

In one embodiment, the cell is an *ex vivo* cell.

The cell may be a cell that is not directly taken from a subject or multicellular organism.

In one embodiment, the cell is an *in vivo* cell.

### Therapeutic and non-therapeutic methods and uses

The methods, polypeptides, binding molecules, nucleic acids, expression cassettes, vectors, and cells of the invention have therapeutic and non-therapeutic utility.

In one aspect, there is provided the binding molecule, the polypeptide, the nucleic acid, the expression cassette, the vector, or the cell of the invention for use in a method of treating a disease.

As used herein treatment is intended to include therapeutic interventions that ameliorate a disease as well as curative treatments. Further, prophylactic use is also encompassed, such that said treatment may include treating a subject that is susceptible to a disease or is otherwise showing signs of progression towards a disease state without necessarily having symptoms of the disease or a clinical diagnosis of the disease.

In one embodiment, there is provided the binding molecule of the invention for use in a method of treating a disease.

In one embodiment, there is provided the polypeptide of the invention for use in a method of treating a disease.

In one embodiment, there is provided the nucleic acid of the invention for use in a method of treating a disease.

In one embodiment, there is provided expression cassette of the invention for use in a method of treating a disease.

In one embodiment, there is provided the vector of the invention for use in a method of treating a disease.

In one embodiment, there is provided the cell of the invention for use in a method of treating a disease.

In one aspect, there is provided a method of treating a disease comprising administering the binding molecule, the polypeptide, the nucleic acid, the expression cassette, the vector, or the cell of the invention to a patient in need thereof.

In one embodiment, there is provided a method of treating a disease comprising administering the binding molecule, the polypeptide, the nucleic acid, the expression cassette, or the vector to a patient in need thereof.

In one embodiment, there is provided a method of treating a disease comprising administering the binding molecule of the invention to a patient in need thereof.

In one embodiment, there is provided a method of treating a disease comprising administering the polypeptide of the invention to a patient in need thereof.

In one embodiment, there is provided a method of treating a disease comprising administering the nucleic acid of the invention to a patient in need thereof.

In one embodiment, there is provided a method of treating a disease comprising administering the expression cassette of the invention to a patient in need thereof.

In one embodiment, there is provided a method of treating a disease comprising administering the vector of the invention to a patient in need thereof.

In one embodiment, there is provided a method of treating a disease comprising administering the cell of the invention to a patient in need thereof.

In one embodiment, said administering is administering to a cell of said patient.

In one embodiment, there is provided a method of treating a disease comprising administering the cell of the invention to a patient in need thereof.

In one aspect, there is provided use of the binding molecule, the polypeptide, the nucleic acid, the expression cassette, the vector, or the cell of the invention for the manufacture of a medicament for use in the treatment of a disease.

In one embodiment, there is provided use of the binding molecule of the invention for the manufacture of a medicament for use in the treatment of a disease.

In one embodiment, there is provided use of the polypeptide of the invention for the manufacture of a medicament for use in the treatment of a disease.

In one embodiment, there is provided use of the nucleic acid of the invention for the manufacture of a medicament for use in the treatment of a disease.

In one embodiment, there is provided use of the vector of the invention for the manufacture of a medicament for use in the treatment of a disease.

In one embodiment, there is provided use of expression cassette of the invention for the manufacture of a medicament for use in the treatment of a disease.

In one embodiment, there is provided use of the cell of the invention for the manufacture of a medicament for use in the treatment of a disease.

In one embodiment, there is provided the binding molecule, polypeptide, nucleic acid, expression cassette, vector, or cell of the invention for use in a method of treating a disease, wherein the disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.

In one embodiment, there is provided a method of treating a disease comprising administering the binding molecule, polypeptide, nucleic acid, expression cassette, vector, or cell of the invention to a patient in need thereof, wherein said disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.

In one embodiment, there is provided use of the binding molecule, polypeptide, nucleic acid, expression cassette, vector, or cell of the invention for the manufacture of a medicament for use in the treatment of a disease wherein said disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.

In one embodiment of the methods and uses herein, the disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.

In one embodiment,
(i) the respiratory disease or disorder is selected from the group consisting of: asthma, chronic obstructive pulmonary disease (COPD), bronchospasm caused by COPD, bronchial asthma, and emphysema;
(ii) the immune disease or disorder is selected from the group consisting of: rheumatoid arthritis (RA), systemic lupus erythematosus (SLE), multiple sclerosis (MS), myasthenia gravis (MG), and Grave's disease;
(iii) the cancer is selected from the group consisting of: lung cancer, breast cancer, prostate cancer, and skin cancer; and
(iv) the cardiovascular disease or disorder is hyperkalemia, or vascular extravasation.

In one embodiment of the methods and uses herein, the disease is a respiratory disease or disorder.

In one embodiment of the methods and uses herein, the disease is a respiratory disease or disorder selected from the group consisting of: asthma, chronic obstructive pulmonary disease (COPD), bronchospasm caused by COPD, bronchial asthma, and emphysema.

In a preferred embodiment, the disease is asthma.

In a preferred embodiment, the disease is COPD.

In one embodiment, the treatment is for bronchospasm.

In one embodiment of the methods and uses herein, the disease is an immune disease or disorder.

In one embodiment of the methods and uses herein, the disease is an immune disease or disorder selected from the group consisting of: rheumatoid arthritis (RA), systemic lupus erythematosus (SLE), multiple sclerosis (MS), myasthenia gravis (MG), and Grave's disease.

In one embodiment of the methods and uses herein, the disease is a cardiovascular disease or disorder.

In one embodiment of the methods and uses herein, the disease is cardiovascular disease or disorder selected from the group consisting of: hyperkalemia, or vascular extravasation.

In one embodiment of the methods and uses herein, the disease is cancer.

In one embodiment of the methods and uses herein, the disease is a cancer associated with β₂AR activity.

In one embodiment of the methods and uses herein, the disease is cancer and is selected from the group consisting of: lung cancer, breast cancer, prostate cancer, and skin cancer.

In some embodiments, the methods and uses of the invention may be considered therapeutic.

In some embodiments, the methods and uses of the invention may be considered non-therapeutic or cosmetic.

In instances where the methods and uses of the invention may be considered non-therapeutic, the patient may be considered to be a subject.

In some embodiments, the methods and uses of the invention, the subject has no known disease or disorder.

In the present application, lists of features preceded with the phrase "at least one of" mean that any one or more of those features can be provided either individually or in combination. For example, "at least one of: [A], [B] and [C]" encompasses any of the following options: A alone (without B or C), B alone (without A or C), C alone (without A or B), A and B in combination (without C), A and C in combination (without B), B and C in combination (without A), or A, B and C in combination.

Although illustrative embodiments of the invention have been described in detail herein with reference to the accompanying drawings, it is to be understood that the invention is not limited to those precise embodiments, and that various changes and modifications can be effected therein by one skilled in the art without departing from the scope of the invention as defined by the appended claims.

### EXAMPLES

### Example 1

Here, we employ a multidimensional deep mutational scanning (DMS) approach to measure the functional effects of nearly all single and thousands of double amino acid mutations in the β₂-adrenergic receptor (β₂AR), a well-studied class A GPCR.

One possible approach is to use DMS to characterize surface expression, ligand binding, or signalling of human GPCRs individually. However, only measuring one component of protein function limits the understanding of the full impact of mutations. Simultaneously measuring the effects of thousands of variants on surface expression and signalling through three transducers representing the two major signal pathways of GPCRs enables an unprecedented view into the functional architecture of the receptor.

One strength of our approach is that the readout is receptor-proximal, abrogating the contribution of cellular signal amplification or feedback.

### Materials and Methods

### Plasmids

Plasmids encoding SPARK2 components were used to generated new plasmids using Gibson cloning. attB-HA-b2AR-eLOV-TEVcs-Gal4-UAS-Citrine encodes the receptor fusion followed by the bGH poly(A) signal, then the UAS sequence followed by Citrine fluorescent protein. A 26-nucleotide barcode was inserted in the 3' UTR of the Citrine sequence. To make cloning more efficient, the fusion coding sequence from the NanoLuc-15aa linker-βarrestin2-HA-GS linker-TEVp plasmid from the pAAV vector (which contains GC-rich inverted terminal repeats) was transferred to the pUC19 vector. Site directed mutagenesis was then used to introduce the S153N variant to TEV, generating uTEV1Δ). Versions of this plasmid, with Nb80 and miniGₛ sequence instead of β-Arrestin, were then made. The miniGₛ vector was also modified so that uTEV1Δ was fused N-terminally (NanoLuc-15aa linker-GS linker-TEVp-miniGₛ).

### Saturation mutagenesis library creation

Six pools of mutation-bearing oligonucleotides were designed, each pool representing mutated coding sequence for 66-70 amino acids of the full-length (413 amino acids) β₂AR with flanking wild-type sequence for amplification from the pool (using primers dialout_tile[1-6]_[F/R]). For each pool, primers were designed to amplify the whole plasmid except for the mutated segment (using primers b2AR_satmut tile[1-6]_[F/R]), then used Gibson assembly to introduce the mutagenic oligos. Oligos were designed to encode all possible single amino acid changes (using the human optimized codon for each alternative amino acid), all premature stop codons, and all synonymous codons when possible (using the most optimal codon, excluding the wild-type codon).

Two sets of double mutants were also designed. First, a set of double mutants aimed at amino acids that are in physical contact in either the active state (PDB: 3SN6), the inactive state (PDB: 2RH1), or both. These contacts were filtered to include only those that were within the boundaries of a single pool, which led to 62 active state-specific and 131 inactive state-specific residue pairs. As a comparison group, 76 residue pairs that are in physical contact in both the active and inactive structure were mutated. For each pair, each residue was mutated to alanine (or glycine if the wild-type was alanine) and also the amino acids were swapped (for example if the wild-type residues were serine and leucine, they were mutated to leucine and serine). Finally, within tile 2, all possible double alanine mutations were made (or glycine if the wild-type was alanine). Oligos were ordered as 250 nucleotides in a 12,000 oligo pool from Twist Bioscience.

### Barcode integration and variant-barcode association

10 µg of the attB-HA-b2AR-eLOV-TEVcs-Gal4-UAS-Citrine saturation mutagenesis plasmid was digested with 80 units Spel-HF for 1.5 hours at 37°, then added 40 more units of Spel-HF and incubated 1.5 more hours at 37°. Then the cut band was gel purified and the gel purification product was digested again with 80 units of Spel-HF for 3 hours. Then, the barcode construct (oTLM_097) was made double stranded by doing one cycle of PCR with oTLM_098 as the primer. Double stranded barcode was incorporated into the cut plasmid backbone by Gibson assembly and transformed into electrocompetent bacteria. Following recovery in SOC media, 0.1% of the transformation was plated to estimate the number of clones, and the rest was inoculated into LB media with ampicillin for overnight growth. Estimated number of clones was 96,000. In the morning the bacteria was pelleted and plasmid was midi-prepped (Qiagen Plasmid Plus Midi Kit).

PacBio long read sequencing was used to associate each variant with the barcode. The plasmid pool was digested with BsiWI and Ncol restriction enzymes to release the coding sequence and barcode and SMRT bell adapters were ligated according to manufacturer's instructions. The library was sequenced on a PacBio Sequel IIe. Alignparse was used to align reads, quality filter, and call sequences for each barcode.

### Landing pad integration

HEK 293T LLP-iCasp9-Blast cells were grown in DMEM with GlutaMAX (Gibco), with Tetracyline-free fetal bovine serum. 11 million cells were plated onto a T175 flask. The next day, transfection was done with Lipofectamine with 13.5 µg of pCAG-NLS-HA-Bxb1 and 13.5 µg of the mutated and barcoded attB-HA-b2AR-eLOV-TEVcs-Gal4-UAS-Citrine. The cells were allowed to incubate for two days. Then, the media was removed and replaced with media containing 2 µg/mL doxycycline and incubated for one day. Then, the media was changed for media containing 2 µg/mL doxycycline and 10 nM rimiducid (MedChemExpress) and incubated for one day. Then media was changed to include just 2 µg/mL doxycycline and outgrowth of cells was allowed for -one week, passaging cells when they reached 90% confluency.

### Signalling experiment

10 million cells were plated onto each T175 flask and allowed to incubate overnight. The next day, the cells were transfected with 36 µg of transducer plasmids (pUC19-NanoLuc-β-arrestin-uTEVp1, pUC19-NanoLuc-Nb80-uTEVp1, or pUC19-NanoLuc-uTEVp1-miniGₛ), using Lipofectamine 3000 (Invitrogen) and allowed to incubate overnight. The next morning, cells were treated with media containing furimazine and either drug or DMSO: [7.295 mL DMEM, 46.875 µL furimazine, 150 µL 1M HEPES, and 7.5 µL 10 mM isoproterenol or DMSO]. Cells were incubated in the dark (covered with aluminum foil) at 37° for 24 hours. Then, cells were lifted with Trypsin-EDTA, pelleted and stored in -80 until RNA extraction. For the Gal4 maximum stimulation condition, after 10 million cells were plated and grown overnight, 36 µg of pUC19-CMV-Gal4 was transfected with Lipofectamine 3000. The cells were allowed to incubate for 30 hours, then cells were pelleted and stored in -80.

### RNA isolation and sequencing library preparation

Cell pellets were thawed and resuspended in buffer RLT with added beta-mercaptoethanol. Then, they were homogenized by centrifuging through QlAshredder columns. RNA was then purified from the homogenate with RNeasy Midi-prep kit (Qiagen) and eluted with 400 µL of water. Between 120 and 160 µg of RNA was used as input for reverse transcription reactions (6-8 µg per reaction, 20 reactions per sample) using SuperScript III reverse transcriptase (Invitrogen). Reactions were carried out with 2 pmol of primer (oTLM266) and incubated at 55° for one hour then 75° for 15 minutes. Following this, RNase A was added to a final concentration of 100 µg/mL and reactions incubated at 37° for 30 minutes. Then, reactions were cleaned up with Nucleospin PCR cleanup columns (Macherey Nagel) and eluted in 50 µL of elution buffer. 0.5 µL of the cDNA eluate was used in a qPCR reaction (Q5 High-Fidelity polymerase, New England Biolabs) to determine the number of cycles required to reach near-plateau phase. Then, 40 µL of cDNA was amplified across 4x 50 µL PCR reactions with the program 98° for 30s, followed by 14-16 cycles of [98° for 15s, 64° for 30s, 72° for 30s]. Reactions were column purified and 4% of this was used as input for a second round PCR, which added the rest of the Illumina adapter as well as unique index sequences. The second round PCR was 98° for 30s, followed by 5 cycles of [98° for 15s, 64° for 30s, 72° for 30s]. Reactions were then gel purified and analyzed on TapeStation (Agilent Technologies). Sequencing was done with Illumina NovaSeq with 2x50 paired end reads.

### Surface expression experiment

Cells were cultured in 245x245mm cell culture flasks until -80% confluent. Then, cells were lifted with Trypsin-EDTA, pelleted, supernatant removed, and resuspended in blocking buffer (1% bovine serum albumin in phosphate buffered saline). Cells were counted and 50 M cells (replicate 1 or 2) were transferred to a 5 mL conical tube with 3.5 mL of blocking buffer, and incubated on ice, covered from light, with rocking for 30 minutes. Then, anti-HA antibody (Cell Signalling HA-Tag 6E2 Alexa Fluor 647 Conjugate) was added at dilution of 1:100 and incubated on ice, covered from light, with rocking for 60 more minutes. Then, cells were pelleted, supernatant removed, and resuspended in blocking buffer. Sorting was done on the BD FACSAria instrument. Bins were drawn so that each represented -25% of cells and each bin received 5-8 million cells.

### Surface expression sequencing library preparation

Sorted cells were pelleted and DNA extracted using Qiagen DNeasy Blood & Tissue kit, using 2 columns for each sample. 200 µL elution buffer was used to elute from each column, then eluates for each sample were combined. Then, 320 µL of each sample was used as input for 16x 50 µL PCRs with primers adjacent to the barcode that include partial Illumina adapters and also varying lengths of degenerate bases to increase nucleotide complexity in the first few cycles of the sequencing run (oTLM277-oTLM282). Cycling was 98° for 30s, followed by 25 cycles of [98° for 15s, 64° for 30s, 72° for 30s]. Reactions were column purified and 4% of this was used as input for a second round PCR, which added the rest of the Illumina adapter as well as unique index sequences. The second round PCR was 98° for 30s, followed by 5 cycles of [98° for 15s, 64° for 30s, 72° for 30s]. The products of the second PCR were gel purified and analyzed on TapeStation, then sequenced on Illumina NovaSeq with 2x50 paired end reads.

### Results

Current results: analysis has identified all 8 residues that are known to participate in ligand binding (this is a positive control), as well as several other residues that are involved in binding the transducer or are in conserved motifs known to be important for GPCR signalling.

Table 1 and 2 summarise how modification of residues at different positions can activate or inactivate the G-protein or B-arrestin pathways.

**Table 1: Full data set relating to the mutation of different residues of the β₂AR and the effect of such mutation on the function of the receptor: (+) = activating; (-) = inactivating. For each pathway, the "average residual" indicates the mean of the residuals determined for each mutated variant having a mutation at the specified position. The "FDR pval" refers to the outcome of performing the Mann-Whitney U test followed by FDR correction with the Benjamini / Hochberg method. Sites with pval < 0.1 (10%) are indicated as either activating (if the average residual for that site is positive) or inactivating (if the average residual for that site is negative).**

| **Position** | **Nb80 average residual** | **Nb80 FDR pval** | **Nb80** | **BArr average residual** | **BArr FDR bool** | **BArr** |
|---|---|---|---|---|---|---|
| **2** | 0.009631014 | 0.874724567 | | 0.108400837 | 0.232991686 | |
| **3** | -0.067800887 | 0.397936624 | | -0.020501418 | 0.563342353 | |
| **4** | -0.172570131 | 0.865627388 | | -0.13238365 | 0.743613878 | |
| **5** | 0.032934672 | 0.747084892 | | 0.002419218 | 0.766143486 | |
| **6** | 0.14089395 | 0.125796286 | | 0.183478053 | 0.00802977 | + |
| **7** | 0.095694895 | 0.36294701 | | -0.136385827 | 0.448040001 | |
| **8** | 0.073747697 | 0.26879498 | | -0.071715054 | 0.908741174 | |
| **9** | 0.052617485 | 0.553397872 | | -0.064805679 | 0.858931301 | |
| **10** | 0.020946472 | 0.981256826 | | -0.134061623 | 0.459717782 | |
| **11** | 0.036910281 | 0.906746807 | | 0.099902712 | 0.343674457 | |
| **12** | 0.079475569 | 0.634161702 | | 0.006972309 | 0.277479425 | |
| **13** | 0.061725898 | 0.403212921 | | 0.024987381 | 0.232991686 | |
| **14** | 0.064527829 | 0.348224881 | | 0.090709622 | 0.03260585 | + |
| **15** | 0.252135824 | 0.000620405 | + | 0.193343078 | 0.00802977 | + |
| **16** | 0.035993665 | 0.562472501 | | -0.068836289 | 0.877487893 | |
| **17** | 0.163400234 | 0.048938137 | + | 0.103775138 | 0.217651353 | |
| **18** | 0.069739095 | 0.454653388 | | 0.012180103 | 0.640544124 | |
| **19** | 0.088783283 | 0.389427672 | | 0.04714656 | 0.256846724 | |
| **20** | 0.138955436 | 0.037704582 | + | -0.007257451 | 0.585436608 | |
| **21** | 0.095457719 | 0.218801327 | | -0.070099558 | 0.985128732 | |
| **22** | 0.055230697 | 0.564112241 | | -0.078345499 | 0.947781495 | |
| **23** | -0.006024244 | 0.871473662 | | -0.01597833 | 0.705348163 | |
| **24** | 0.149088459 | 0.210166842 | | 0.163541709 | 0.031330049 | + |
| **25** | 0.073875054 | 0.607350633 | | 0.033838299 | 0.215593993 | |
| **26** | 0.235544365 | 0.005470847 | + | 0.223810768 | 0.000857335 | + |
| **27** | 0.093373268 | 0.346688488 | | 0.042944499 | 0.259355767 | |
| **28** | -0.078744998 | 0.925166466 | | 0.113580667 | 0.016156403 | + |
| **29** | 0.054951849 | 0.981256826 | | 0.106142785 | 0.323251308 | |
| **30** | 0.129604102 | 0.414304668 | | 0.012880816 | 0.891265025 | |
| **31** | -0.043091925 | 0.7428204 | | -0.052034139 | 0.994825402 | |
| **32** | -0.054871962 | 0.634161702 | | 0.009685735 | 0.413750237 | |
| **33** | 0.053357673 | 0.417237849 | | 0.06582139 | 0.198330022 | |
| **34** | -0.025851762 | 0.820648771 | | 0.000873843 | 0.485091166 | |
| **35** | 0.120245101 | 0.131353754 | | 0.058823082 | 0.203073214 | |
| **36** | 0.166061011 | 0.073704819 | + | 0.014078169 | 0.554722532 | |
| **37** | 0.278629775 | 0.005186778 | + | 0.122742152 | 0.092354868 | + |
| **38** | 0.109430164 | 0.191266527 | | 0.034866519 | 0.50405095 | |
| **39** | -0.11613673 | 0.349478115 | | -0.206062766 | 0.100454663 | |
| **40** | 0.141085629 | 0.327902375 | | 0.057646284 | 0.210440824 | |
| **41** | 0.130239437 | 0.667829517 | | -0.042398171 | 0.614250469 | |
| **42** | -0.101653054 | 0.50500422 | | -0.157064059 | 0.567650295 | |
| **43** | 0.153140956 | 0.027149472 | + | -0.168082031 | 0.584957601 | |
| **44** | 0.14116648 | 0.185389861 | | 0.132288811 | 0.056975075 | + |
| **45** | 0.001812235 | 0.852208748 | | -0.026416235 | 0.620227336 | |
| **46** | 0.00528491 | 0.904766637 | | -0.139997944 | 0.459717782 | |
| **47** | -0.040869191 | 0.879619867 | | -0.23773766 | 0.942644016 | |
| **48** | 0.07590501 | 0.185389861 | | -0.106588117 | 0.984212015 | |
| **49** | 0.062949572 | 0.719508333 | | 0.107433339 | 0.031330049 | + |
| **50** | 0.107097304 | 0.125796286 | | 0.093020197 | 0.010942739 | + |
| **51** | -0.078568667 | 0.570338196 | | -0.126047537 | 0.7416258 | |
| **52** | 0.008963128 | 0.805322941 | | 0.016303886 | 0.468155463 | |
| **53** | -0.005437894 | 0.785323287 | | -0.175982495 | 0.146061179 | |
| **54** | -0.176191893 | 0.271928462 | | -0.384353685 | 0.006665598 | - |
| **55** | -0.040595988 | 0.800901902 | | -0.156818613 | 0.426873611 | |
| **56** | 0.0413604 | 0.706406253 | | -0.135137113 | 0.361589216 | |
| **57** | 0.063543312 | 0.363456093 | | -0.235506894 | 0.635906221 | |
| **58** | 0.110232765 | 0.357941977 | | -7.84E-05 | 0.554722532 | |
| **59** | -0.000744203 | 0.820648771 | | -0.08855001 | 0.880029838 | |
| **60** | 0.061765507 | 0.801510533 | | -0.06301789 | 0.419756845 | |
| **61** | 0.047312527 | 0.977628523 | | 0.040630907 | 0.608421883 | |
| **62** | 0.069799087 | 0.557154244 | | -0.034507935 | 0.743613878 | |
| **63** | -0.003235612 | 0.742335158 | | -0.048543727 | 0.886140023 | |
| **64** | 0.186039585 | 0.084808075 | + | 0.098729275 | 0.586243154 | |
| **65** | -0.052972569 | 0.634161702 | | -0.149905681 | 0.232991686 | |
| **66** | -0.094325648 | 0.436057266 | | -0.254164869 | 0.180816857 | |
| **67** | -0.465817593 | 0.097676533 | - | -0.434157412 | 0.757532626 | |
| **68** | -0.060575433 | 0.637637687 | | -0.063132732 | 0.862063174 | |
| **69** | 0.03292979 | 0.637637687 | | -0.124536886 | 0.705348163 | |
| **70** | 0.001185514 | 0.885480466 | | -0.089920321 | 0.857982429 | |
| **71** | -0.061957219 | 0.372262053 | | -0.307467975 | 0.001502744 | - |
| **72** | -0.124942163 | 0.125796286 | | -0.199226787 | 0.114244692 | |
| **73** | 0.048870034 | 0.400453483 | | 0.023674482 | 0.259355767 | |
| **74** | 0.110965217 | 0.14709442 | | -0.082592012 | 0.926399053 | |
| **75** | -0.225808947 | 0.234860192 | | -0.291634952 | 0.053440265 | - |
| **76** | -0.15289816 | 0.063257315 | - | -0.159455615 | 0.210440824 | |
| **77** | -0.049807793 | 0.762862026 | | -0.076041454 | 0.932450668 | |
| **78** | -0.184040088 | 0.102319058 | | -0.252121525 | 0.053440265 | - |
| **79** | -0.309569064 | 0.002903634 | - | -0.393374028 | 0.000997668 | - |
| **80** | -0.129880245 | 0.125796286 | | -0.17887713 | 0.097776103 | - |
| **81** | -0.05382905 | 0.607350633 | | -0.023979512 | 0.575501672 | |
| **82** | -0.425532608 | 0.000620405 | - | -0.408459542 | 0.000917565 | - |
| **83** | -0.013198232 | 0.808125751 | | -0.120828526 | 0.565667622 | |
| **84** | -0.083653258 | 0.242734594 | | -0.136968619 | 0.426873611 | |
| **85** | 0.121344855 | 0.095563938 | + | -0.003894711 | 0.661592996 | |
| **86** | -0.074188189 | 0.653387266 | | -0.214262029 | 0.119859805 | |
| **87** | 0.304801664 | 0.000319899 | + | 0.230692984 | 0.000825192 | + |
| **88** | -0.052496994 | 0.406768585 | | -0.154575614 | 0.195710281 | |
| **89** | 0.130208065 | 0.150667759 | | -0.011918405 | 0.586243154 | |
| **90** | 0.11917593 | 0.141791622 | | -0.013909547 | 0.614250469 | |
| **91** | 0.178857769 | 0.048938137 | + | 0.043682642 | 0.232991686 | |
| **92** | 0.100694558 | 0.340793902 | | 0.065503473 | 0.146061179 | |
| **93** | -0.002358668 | 0.996021164 | | -0.016127508 | 0.520298349 | |
| **94** | 0.035576839 | 0.761015436 | | 0.047141147 | 0.065467217 | + |
| **95** | 0.143812268 | 0.098650268 | + | 0.069475903 | 0.070168937 | + |
| **96** | 0.147169272 | 0.07769698 | + | 0.022086111 | 0.186030281 | |
| **97** | 0.031207415 | 0.742609493 | | -0.099969549 | 0.847189569 | |
| **98** | 0.063178226 | 0.414997901 | | 0.028076592 | 0.232414544 | |
| **99** | 0.126084325 | 0.136895315 | | -0.212318558 | 0.043364447 | - |
| **100** | -0.018324088 | 0.820648771 | | -0.089099702 | 0.723211223 | |
| **101** | 0.296362453 | 0.000580408 | + | 0.119273705 | 0.085472831 | + |
| **102** | 0.151022392 | 0.016584956 | + | -0.036653429 | 0.675516664 | |
| **103** | -0.021293237 | 0.866478479 | | -0.10339728 | 0.692844716 | |
| **104** | -0.026776384 | 0.797921799 | | -0.172472938 | 0.16776822 | |
| **105** | 0.183490452 | 0.007163738 | + | 0.061185749 | 0.426873611 | |
| **106** | -0.255661571 | 7.54E-05 | - | -0.255654049 | 0.01024087 | - |
| **107** | 0.053600008 | 0.602446123 | | -0.038911602 | 0.708045787 | |
| **108** | -0.073534164 | 0.388446595 | | -0.192389655 | 0.332936213 | |
| **109** | -0.061642499 | 0.304229319 | | -0.213468944 | 0.154777511 | |
| **110** | -0.028287031 | 0.781765718 | | -0.136624535 | 0.601559231 | |
| **111** | -0.092130171 | 0.491736512 | | -0.266783876 | 0.056975075 | - |
| **112** | 0.046594469 | 0.820648771 | | -0.009364251 | 0.485091166 | |
| **113** | -0.881056983 | 2.53E-09 | - | -0.84689138 | 2.32E-08 | - |
| **114** | -0.812428524 | 2.53E-09 | - | -0.770764804 | 2.34E-08 | - |
| **115** | -0.094734652 | 0.196424338 | | -0.156545446 | 0.277479425 | |
| **116** | -0.045645931 | 0.684815496 | | -0.129829101 | 0.552558038 | |
| **117** | -0.647257564 | 6.46E-06 | - | -0.599580553 | 1.17E-06 | - |
| **118** | -0.515943793 | 2.09E-07 | - | -0.557355114 | 6.62E-07 | - |
| **119** | -0.22044319 | 0.00182403 | - | -0.282765527 | 0.010942739 | - |
| **120** | -0.134601157 | 0.532876562 | | -0.134700483 | 0.932450668 | |
| **121** | -0.407662464 | 0.005930826 | - | -0.493588493 | 0.000800217 | - |
| **122** | -0.083760637 | 0.417237849 | | -0.102761916 | 0.830645846 | |
| **123** | 0.105143276 | 0.227210823 | | 0.063749515 | 0.105861778 | |
| **124** | 0.272630069 | 0.007003784 | + | 0.284647411 | 0.006665598 | + |
| **125** | -0.041638196 | 0.885480466 | | -0.139054827 | 0.365626299 | |
| **126** | -0.099026166 | 0.21740206 | | -0.196646985 | 0.055066942 | - |
| **127** | -0.448081225 | 0.000649376 | - | -0.530220245 | 8.41E-06 | - |
| **128** | -0.253520239 | 0.071841628 | - | -0.260308438 | 0.069960612 | - |
| **129** | -0.190308338 | 0.082472597 | - | -0.342496153 | 0.00546976 | - |
| **130** | 0.134637852 | 0.125796286 | | 0.104458763 | 0.067767253 | + |
| **131** | -0.491913561 | 1.13E-06 | - | -0.364122796 | 0.008537532 | - |
| **132** | 0.065818948 | 0.820648771 | | 0.002258551 | 0.659906428 | |
| **133** | -0.078833062 | 0.454653388 | | -0.176802247 | 0.232991686 | |
| **134** | -0.0236947 | 0.820648771 | | -0.023145097 | 0.581090726 | |
| **135** | -0.103006641 | 0.196424338 | | -0.14399358 | 0.162567779 | |
| **136** | -0.10033082 | 0.157469246 | | -0.061439485 | 0.828272935 | |
| **137** | -0.048946134 | 0.850035528 | | -0.079663678 | 0.96053975 | |
| **138** | -0.734542255 | 2.53E-09 | - | -0.606166612 | 5.88E-08 | - |
| **139** | -0.621486391 | 2.17E-08 | - | -0.477917009 | 1.60E-05 | - |
| **140** | -0.069609474 | 0.346688488 | | -0.126248812 | 0.586243154 | |
| **141** | -0.21308654 | 0.003581786 | - | -0.250918176 | 0.042323619 | - |
| **142** | 0.048074406 | 0.603671578 | | 0.032995826 | 0.430142432 | |
| **143** | 0.033976229 | 0.661117196 | | -0.050105642 | 0.814314699 | |
| **144** | -0.006981478 | 0.805527996 | | 0.041203915 | 0.232991686 | |
| **145** | -0.14880676 | 0.020944063 | - | -0.189278615 | 0.110440749 | |
| **146** | -0.041292287 | 0.637595658 | | -0.17691497 | 0.146061179 | |
| **147** | -0.010650202 | 0.912101395 | | -0.091199942 | 0.670372948 | |
| **148** | 0.040298206 | 0.785323287 | | -0.05173398 | 0.932450668 | |
| **149** | -0.049362757 | 0.50500422 | | -0.127931479 | 0.323251308 | |
| **150** | 0.004223878 | 0.970730179 | | -0.115399589 | 0.552558038 | |
| **151** | -0.068409639 | 0.340949232 | | -0.051706748 | 0.907728354 | |
| **152** | 0.030547586 | 0.603671578 | | -0.026854621 | 0.670372948 | |
| **153** | -0.112681664 | 0.142069517 | | -0.191746995 | 0.090238616 | - |
| **154** | 0.020156415 | 0.874724567 | | -0.156821703 | 0.232414544 | |
| **155** | 0.019995395 | 0.781765718 | | -0.045490637 | 0.932450668 | |
| **156** | -0.032810547 | 0.956001471 | | -0.09457305 | 0.982992221 | |
| **157** | -0.02640844 | 0.917638867 | | -0.11458471 | 0.581433638 | |
| **158** | -0.073609948 | 0.50500422 | | -0.180936395 | 0.142596955 | |
| **159** | -0.104062377 | 0.276357245 | | -0.121831665 | 0.563342353 | |
| **160** | -0.074568869 | 0.397936624 | | -0.239579232 | 0.066391048 | - |
| **161** | -0.215639316 | 0.007133777 | - | -0.15640431 | 0.09686508 | - |
| **162** | -0.02267458 | 0.885480466 | | -0.136791789 | 0.586243154 | |
| **163** | 0.003715012 | 0.808125751 | | -0.08097991 | 0.776399848 | |
| **164** | -0.212753792 | 0.065342812 | - | -0.26735749 | 0.024521505 | - |
| **165** | -0.258988684 | 0.00074035 | - | -0.287594295 | 0.030016423 | - |
| **166** | -0.145441953 | 0.196424338 | | -0.265188664 | 0.00802977 | - |
| **167** | -0.0635834 | 0.634161702 | | -0.19228383 | 0.119859805 | |
| **168** | 0.283379526 | 0.001906078 | + | 0.047510957 | 0.500100299 | |
| **169** | 0.008460999 | 0.850035528 | | -0.162311748 | 0.278456276 | |
| **170** | 0.111099934 | 0.129398162 | | 0.045809584 | 0.235533561 | |
| **171** | -0.01126605 | 0.999556746 | | -0.077527932 | 0.869916987 | |
| **172** | 0.028350272 | 0.80845791 | | -0.047768869 | 0.994825402 | |
| **173** | 0.063270106 | 0.479013202 | | -0.161676067 | 0.299634486 | |
| **174** | 0.131741449 | 0.037494106 | + | -0.04738635 | 0.743613878 | |
| **175** | -0.029211057 | 0.885480466 | | -0.099356023 | 0.659906428 | |
| **176** | -0.000701474 | 0.951976827 | | -0.146475719 | 0.232414544 | |
| **177** | -0.06717021 | 0.562737565 | | -0.087942481 | 0.976785668 | |
| **178** | 0.028325418 | 0.722460623 | | 0.006031529 | 0.601559231 | |
| **179** | 0.078401287 | 0.175769229 | | 0.035061137 | 0.232991686 | |
| **180** | -0.000909304 | 0.917638867 | | -0.015279115 | 0.761514887 | |
| **181** | 0.063773478 | 0.36294701 | | -0.268352356 | 0.008537532 | - |
| **182** | 0.035414575 | 0.553397872 | | 0.097039832 | 0.031330049 | + |
| **183** | 0.084940447 | 0.218801327 | | -0.080442499 | 0.907728354 | |
| **184** | -0.148831016 | 0.033547205 | - | -0.32980894 | 0.034585821 | - |
| **185** | 0.233896818 | 0.001906078 | + | -0.060918143 | 0.863337854 | |
| **186** | 0.092291632 | 0.136895315 | | 0.035434397 | 0.231165625 | |
| **187** | 0.198019393 | 0.005868516 | + | 0.099249842 | 0.024521505 | + |
| **188** | 0.039519216 | 0.357597198 | | -0.115984758 | 0.620227336 | |
| **189** | 0.061466181 | 0.436057266 | | -0.022041464 | 0.706696949 | |
| **190** | -0.132014143 | 0.041737202 | - | -0.20859041 | 0.056975075 | - |
| **191** | -0.307095165 | 8.46E-06 | - | -0.234737872 | 0.021291915 | - |
| **192** | 0.007930605 | 0.80943211 | | -0.096985944 | 0.692844716 | |
| **193** | -0.340507868 | 0.000237355 | - | -0.438059157 | 0.000120211 | - |
| **194** | -0.027305533 | 0.879619867 | | -0.133159021 | 0.659906428 | |
| **195** | -0.00871577 | 0.925166466 | | -0.093219501 | 0.932450668 | |
| **196** | 0.085062471 | 0.241372717 | | -0.123324672 | 0.51062036 | |
| **197** | 0.010724855 | 0.996021164 | | -0.205590167 | 0.160852715 | |
| **198** | 0.026286094 | 0.874724567 | | -0.002672061 | 0.488772114 | |
| **199** | -0.235709638 | 0.011425379 | - | -0.360815634 | 0.001128342 | - |
| **200** | -0.145973072 | 0.153500817 | | -0.292478142 | 0.02001593 | - |
| **201** | 0.07480314 | 0.346688488 | | -0.115749569 | 0.794480825 | |
| **202** | 0.074930965 | 0.19592768 | | -0.037319209 | 0.640544124 | |
| **203** | -0.682899193 | 1.74E-05 | - | -0.669760755 | 4.02E-05 | - |
| **204** | -0.718571193 | 2.36E-07 | - | -0.648332911 | 5.05E-07 | - |
| **205** | 0.007193996 | 0.603671578 | | -0.088437063 | 0.915079766 | |
| **206** | -0.009787443 | 0.80943211 | | -0.039054143 | 0.58041849 | |
| **207** | -0.651526609 | 1.97E-07 | - | -0.635496339 | 3.73E-08 | - |
| **208** | -0.416155568 | 0.001382382 | - | -0.424390074 | 0.000344392 | - |
| **209** | -0.171693511 | 0.011425379 | - | -0.326549628 | 0.0021083 | - |
| **210** | 0.032667337 | 0.634161702 | | -0.049361692 | 0.743613878 | |
| **211** | -0.105707678 | 0.304229319 | | -0.259872859 | 0.03260585 | - |
| **212** | 0.015194953 | 0.820648771 | | -0.093051756 | 0.601559231 | |
| **213** | 0.115291129 | 0.118111669 | | 0.004335394 | 0.553249055 | |
| **214** | 0.045464806 | 0.742609493 | | -0.082129588 | 0.9738841 | |
| **215** | -0.080210298 | 0.637595658 | | -0.185396329 | 0.415146854 | |
| **216** | -0.00820803 | 0.820648771 | | -0.184821299 | 0.314293654 | |
| **217** | 0.018060072 | 0.820648771 | | -0.067171615 | 0.743232268 | |
| **218** | 0.252917343 | 0.00343984 | + | 0.188729177 | 0.024046623 | + |
| **219** | -0.292030718 | 0.000758871 | - | -0.350049042 | 0.003012396 | - |
| **220** | -0.063613158 | 0.557373629 | | -0.07069286 | 0.743613878 | |
| **221** | 0.037435 | 0.996021164 | | -0.026400855 | 0.880029838 | |
| **222** | -0.07100329 | 0.956001471 | | -0.154191524 | 0.620227336 | |
| **223** | -0.03096585 | 0.820648771 | | -0.052138801 | 0.932450668 | |
| **224** | -0.007652956 | 0.923939633 | | 0.041081048 | 0.164661371 | |
| **225** | 0.082671827 | 0.346017642 | | 0.117457732 | 0.005537149 | + |
| **226** | 0.060911668 | 0.564112241 | | -0.042933026 | 0.743613878 | |
| **227** | 0.086713081 | 0.141791622 | | -0.07056192 | 0.934787993 | |
| **228** | 0.106727555 | 0.153429029 | | -0.036051621 | 0.776399848 | |
| **229** | 0.082215099 | 0.189854542 | | 0.202412426 | 0.011264144 | + |
| **230** | 0.044081106 | 0.216099507 | | 0.002956756 | 0.210079773 | |
| **231** | 0.089102553 | 0.148657084 | | 0.02707804 | 0.190454057 | |
| **232** | -0.051506705 | 0.637637687 | | 0.054289623 | 0.152039517 | |
| **233** | -0.029380451 | 0.702143296 | | -0.007384057 | 0.557419299 | |
| **234** | 0.135359568 | 0.008291352 | + | 0.039048416 | 0.103561208 | |
| **235** | -0.067300637 | 0.588658606 | | -0.034619675 | 0.488772114 | |
| **236** | 0.193569225 | 0.002262792 | + | 0.121153532 | 0.079106347 | + |
| **237** | 0.057918911 | 0.564112241 | | 0.07296289 | 0.146061179 | |
| **238** | 0.054657374 | 0.61052397 | | 0.047939215 | 0.178879972 | |
| **239** | -0.004494232 | 0.966653972 | | -0.113892463 | 0.675516664 | |
| **240** | 0.090921036 | 0.185389861 | | 0.128971046 | 0.01427697 | + |
| **241** | 0.066999001 | 0.424387761 | | 0.019866721 | 0.232414544 | |
| **242** | 0.007256029 | 0.925166466 | | 0.009318876 | 0.43762148 | |
| **243** | -0.029239555 | 0.951976827 | | -0.064331605 | 0.976785668 | |
| **244** | 0.056540711 | 0.603671578 | | -0.004120441 | 0.448040001 | |
| **245** | 0.025543903 | 0.781765718 | | 0.026170898 | 0.256846724 | |
| **246** | 0.112678981 | 0.136528011 | | 0.126350496 | 0.006858995 | + |
| **247** | 0.029400809 | 0.703733042 | | -0.023351499 | 0.620227336 | |
| **248** | 0.019904813 | 0.708832146 | | -0.007595035 | 0.601559231 | |
| **249** | 0.012062979 | 0.820648771 | | -0.002661255 | 0.422567362 | |
| **250** | 0.12868355 | 0.196424338 | | 0.056827001 | 0.123950296 | |
| **251** | 0.040939268 | 0.564112241 | | 0.045129464 | 0.102133965 | |
| **252** | -0.046247525 | 0.703733042 | | -0.047153445 | 0.554280715 | |
| **253** | -0.034636833 | 0.591529018 | | -0.059280545 | 0.880029838 | |
| **254** | 0.062984046 | 0.61052397 | | -0.182688133 | 0.337412388 | |
| **255** | 0.036509213 | 0.653563712 | | 0.069451521 | 0.090640277 | + |
| **256** | -0.117257538 | 0.170680078 | | -0.14053007 | 0.722288996 | |
| **257** | 0.022402884 | 0.797921799 | | -0.025656274 | 0.791154354 | |
| **258** | 0.008983523 | 0.761941715 | | 0.028912007 | 0.162567779 | |
| **259** | -0.039976415 | 0.634161702 | | -0.057031152 | 0.847189569 | |
| **260** | 0.049466675 | 0.661117196 | | -0.023874138 | 0.675516664 | |
| **261** | 0.027272578 | 0.64626033 | | 0.039278573 | 0.152039517 | |
| **262** | -0.007354567 | 0.934104808 | | 0.01171907 | 0.459584462 | |
| **263** | -0.059450452 | 0.406768585 | | -0.139813687 | 0.880029838 | |
| **264** | -0.061554742 | 0.564112241 | | -0.183303172 | 0.908741174 | |
| **265** | -0.008639242 | 0.930825459 | | 0.03724674 | 0.146061179 | |
| **266** | 0.012830486 | 0.591529018 | | 0.027978009 | 0.214190766 | |
| **267** | -0.028650553 | 0.790061082 | | 0.043420929 | 0.164286366 | |
| **268** | 0.424189701 | 8.77E-07 | + | 0.455950714 | 6.62E-07 | + |
| **269** | 0.0960195 | 0.097676533 | + | 0.054204119 | 0.150460348 | |
| **270** | -0.035642012 | 0.722460623 | | 0.016836572 | 0.214190766 | |
| **271** | -0.043003661 | 0.704276418 | | -0.055747765 | 0.84506029 | |
| **272** | 0.152654628 | 0.065342812 | + | 0.17213619 | 0.001336573 | + |
| **273** | 0.00524339 | 0.820648771 | | -0.069812109 | 0.485091166 | |
| **274** | 0.112551506 | 0.40493011 | | 0.063924752 | 0.488772114 | |
| **275** | -0.11751457 | 0.280464531 | | -0.236809545 | 0.106261494 | |
| **276** | 0.148066672 | 0.097676533 | + | 0.164686412 | 0.018133021 | + |
| **277** | 0.06257989 | 0.406768585 | | 0.139050015 | 0.006665598 | + |
| **278** | -0.275088558 | 0.003581786 | - | -0.243096809 | 0.035450217 | - |
| **279** | -0.066331015 | 0.485418448 | | -0.189686185 | 0.235533561 | |
| **280** | -0.142950085 | 0.047602456 | - | -0.345403248 | 0.002563869 | - |
| **281** | -0.07041744 | 0.618115139 | | -0.059382225 | 0.946708928 | |
| **282** | -0.181043001 | 0.118111669 | | -0.180796779 | 0.119859805 | |
| **283** | 0.169974036 | 0.008291352 | + | 0.066766996 | 0.095625905 | + |
| **284** | 0.08396433 | 0.219446045 | | -0.009953354 | 0.753207356 | |
| **285** | 0.114788398 | 0.097676533 | + | -0.076563174 | 0.899306511 | |
| **286** | -0.393915309 | 3.86E-06 | - | -0.476203316 | 4.99E-05 | - |
| **287** | 0.082511113 | 0.244516578 | | 0.002684382 | 0.51062036 | |
| **288** | 0.018800176 | 0.911083474 | | -0.135385291 | 0.595859137 | |
| **289** | -0.562629523 | 3.51E-08 | - | -0.503333688 | 4.07E-05 | - |
| **290** | -0.471130802 | 3.86E-06 | - | -0.53553199 | 1.34E-06 | - |
| **291** | 0.099310453 | 0.218801327 | | -0.021613723 | 0.628566421 | |
| **292** | -0.024175613 | 0.820648771 | | -0.143121514 | 0.300974601 | |
| **293** | -0.307025933 | 0.000152299 | - | -0.411222908 | 1.75E-05 | - |
| **294** | -0.179696654 | 0.048938137 | - | -0.292634622 | 0.00802977 | - |
| **295** | 0.174866602 | 0.044517118 | + | 0.00644362 | 0.283445429 | |
| **296** | 0.009006524 | 0.996021164 | | -0.074237532 | 0.908741174 | |
| **297** | -0.034647192 | 0.72662883 | | 0.023004139 | 0.210440824 | |
| **298** | -0.01813823 | 0.925166466 | | -0.024436694 | 0.635906221 | |
| **299** | 0.001662578 | 0.956001471 | | 0.008177199 | 0.554722532 | |
| **300** | 0.026288017 | 0.608428024 | | -0.015947855 | 0.554722532 | |
| **301** | -0.0041738 | 0.820648771 | | -0.063221344 | 0.851531999 | |
| **302** | -0.03945618 | 0.634161702 | | -0.0745342 | 0.874382596 | |
| **303** | -0.011897934 | 0.915127779 | | -0.063066417 | 0.693867875 | |
| **304** | -0.087605108 | 0.389427672 | | -0.122451976 | 0.554722532 | |
| **305** | -0.001910795 | 0.820648771 | | -0.060465989 | 0.996655458 | |
| **306** | 0.148404798 | 0.171985973 | | 0.004683603 | 0.828256852 | |
| **307** | 0.00147501 | 0.964160505 | | -0.040735878 | 0.753207356 | |
| **308** | -0.07222882 | 0.414997901 | | -0.242267637 | 0.044466818 | - |
| **309** | -0.010708265 | 0.871473662 | | -0.013153822 | 0.631405218 | |
| **310** | 0.042708705 | 0.567095768 | | -0.002442207 | 0.640544124 | |
| **311** | -0.031235218 | 0.634161702 | | -0.14087471 | 0.315490055 | |
| **312** | -0.577620576 | 2.17E-08 | - | -0.600844875 | 2.34E-08 | - |
| **313** | 0.325713226 | 0.000199838 | + | 0.212360427 | 0.007529713 | + |
| **314** | 0.114886171 | 0.348224881 | | 0.104373593 | 0.026038519 | + |
| **315** | -0.247295988 | 0.000379483 | - | -0.247117139 | 0.019221383 | - |
| **316** | -0.159283175 | 0.125796286 | | -0.278031102 | 0.00802977 | - |
| **317** | 0.125101166 | 0.159994153 | | 0.054877087 | 0.137559665 | |
| **318** | -0.21145839 | 0.065342812 | - | -0.270321215 | 0.044448915 | - |
| **319** | -0.074493123 | 0.642588562 | | -0.117367746 | 0.635906221 | |
| **320** | -0.024252535 | 0.835071109 | | -0.147680473 | 0.517367658 | |
| **321** | -0.111397616 | 0.416468023 | | -0.224173751 | 0.114244692 | |
| **322** | -0.517317086 | 2.19E-05 | - | -0.548039555 | 1.32E-06 | - |
| **323** | 0.227047813 | 0.005455427 | + | 0.065794569 | 0.085472831 | + |
| **324** | 0.022019496 | 0.852208748 | | -0.085770311 | 0.926399053 | |
| **325** | -0.429492177 | 3.90E-06 | - | -0.457561268 | 1.58E-05 | - |
| **326** | -0.620983676 | 1.08E-05 | - | -0.506752411 | 2.74E-05 | - |
| **327** | -0.445986942 | 0.010386189 | - | -0.436622182 | 0.00546976 | - |
| **328** | -0.232118807 | 0.002561708 | - | -0.30171357 | 0.004367657 | - |
| **329** | -0.486664828 | 0.000235112 | - | -0.502468782 | 0.000263752 | - |
| **330** | -0.08415151 | 0.136895315 | | -0.259321562 | 0.00802977 | - |
| **331** | 0.04163787 | 0.556812035 | | -0.001940469 | 0.459584462 | |
| **332** | -0.021736931 | 0.785323287 | | -0.165882374 | 0.200177817 | |
| **333** | 0.044512504 | 0.533915564 | | -0.019583188 | 0.554722532 | |
| **334** | -0.027292811 | 0.820648771 | | -0.002688665 | 0.601559231 | |
| **335** | 0.082374966 | 0.304229319 | | -0.08667256 | 0.766143486 | |
| **336** | 0.036359671 | 0.7428204 | | -0.069352938 | 0.880029838 | |
| **337** | 0.075344608 | 0.562737565 | | -0.003918844 | 0.554722532 | |
| **338** | -0.033738002 | 0.653056306 | | -0.148047868 | 0.326034799 | |
| **339** | -0.033801377 | 0.703733042 | | -0.122205977 | 0.372367721 | |
| **340** | 0.06837666 | 0.346688488 | | -0.03348311 | 0.635906221 | |
| **341** | -0.089370823 | 0.19592768 | | -0.090382332 | 0.946708928 | |
| **342** | -0.008843688 | 0.820648771 | | -0.001038631 | 0.426873611 | |
| **343** | 0.072242403 | 0.487574541 | | 0.012742097 | 0.40076305 | |
| **344** | 0.011319283 | 0.934104808 | | -0.056338072 | 0.981939441 | |
| **345** | 0.086499596 | 0.111981428 | | 0.017316459 | 0.232208843 | |
| **346** | 0.072938983 | 0.284637564 | | 0.079777201 | 0.051011947 | + |
| **347** | 0.046877767 | 0.434168161 | | -0.000914638 | 0.284299995 | |
| **348** | 0.042847795 | 0.313107639 | | 0.024131774 | 0.164198683 | |
| **349** | 0.043510608 | 0.564112241 | | 0.024115095 | 0.315490055 | |
| **350** | 0.013920889 | 0.820648771 | | 0.032711162 | 0.214190766 | |
| **351** | 0.055451713 | 0.340793902 | | 0.089820107 | 0.051002521 | + |
| **352** | 0.10470518 | 0.066514686 | + | 0.044860106 | 0.152039517 | |
| **353** | -0.030827872 | 0.640737686 | | -0.025192543 | 0.616883987 | |
| **354** | -0.037284893 | 0.634161702 | | -0.071215532 | 0.976785668 | |
| **355** | 0.111904085 | 0.115160817 | | 0.09541355 | 0.011675027 | + |
| **356** | -0.004035999 | 0.761015436 | | 0.016141862 | 0.152039517 | |
| **357** | 0.105546864 | 0.417237849 | | 0.129782814 | 0.029236254 | + |
| **358** | -0.02020961 | 0.874724567 | | 0.013766288 | 0.210440824 | |
| **359** | 0.098037948 | 0.115160817 | | -0.005908627 | 0.415146854 | |
| **360** | 0.052018481 | 0.432052469 | | 0.06010203 | 0.077121574 | + |
| **361** | 0.062765396 | 0.406768585 | | 0.037213386 | 0.112775338 | |
| **362** | 0.081453152 | 0.17170514 | | 0.061129596 | 0.053058378 | + |
| **363** | 0.012594633 | 0.871473662 | | 0.023294548 | 0.16776822 | |
| **364** | 0.031594632 | 0.661117196 | | -0.032430444 | 0.659906428 | |
| **365** | 0.01891221 | 0.832380691 | | 0.004511727 | 0.41698105 | |
| **366** | 0.017648969 | 0.753914972 | | 0.046478062 | 0.154777511 | |
| **367** | 0.086321922 | 0.218801327 | | 0.024990559 | 0.157846182 | |
| **368** | 0.005249558 | 0.934104808 | | 0.048955327 | 0.068811163 | + |
| **369** | 0.083833163 | 0.125796286 | | 0.095154692 | 0.010865409 | + |
| **370** | -0.009002388 | 0.968656275 | | -0.01314455 | 0.464600273 | |
| **371** | 0.005794797 | 0.785323287 | | 0.035657436 | 0.173318845 | |
| **372** | 0.047763881 | 0.603671578 | | 0.068282384 | 0.072319896 | + |
| **373** | 0.034996717 | 0.603671578 | | 0.077754613 | 0.026958365 | + |
| **374** | 0.098139805 | 0.141518543 | | 0.125177009 | 0.010942739 | + |
| **375** | 0.127210247 | 0.018240059 | + | 0.145550799 | 0.001391738 | + |
| **376** | 0.071075194 | 0.370280135 | | -0.001888501 | 0.485091166 | |
| **377** | -0.038356311 | 0.820648771 | | -0.052134671 | 0.562829972 | |
| **378** | 0.036701705 | 0.658199492 | | 0.035884633 | 0.186030281 | |
| **379** | 0.061949715 | 0.310925435 | | 0.020632323 | 0.26648005 | |
| **380** | 0.054036234 | 0.40493011 | | 0.014351626 | 0.327578656 | |
| **381** | -0.091765378 | 0.603671578 | | -0.021294329 | 0.595859137 | |
| **382** | 0.038035002 | 0.603671578 | | -0.014522058 | 0.533050481 | |
| **383** | 0.092061638 | 0.284637564 | | 0.074636218 | 0.056975075 | + |
| **384** | 0.082597881 | 0.125796286 | | 0.009419066 | 0.289639997 | |
| **385** | 0.039345327 | 0.603671578 | | 0.022271793 | 0.232414544 | |
| **386** | 0.069362769 | 0.280464531 | | 0.046896531 | 0.073064179 | + |
| **387** | 0.073269858 | 0.634161702 | | 0.047774638 | 0.257481255 | |
| **388** | 0.076402371 | 0.367498904 | | 0.090906868 | 0.040319091 | + |
| **389** | 0.024022746 | 0.726408411 | | -0.03596041 | 0.743613878 | |
| **390** | 0.049520553 | 0.7428204 | | 0.005081888 | 0.686501242 | |
| **391** | 0.033303443 | 0.573179847 | | 0.046475768 | 0.124619529 | |
| **392** | 0.035091971 | 0.61052397 | | 0.017515285 | 0.122757096 | |
| **393** | -0.00075897 | 0.862069626 | | -0.082812962 | 0.900858027 | |
| **394** | -0.025056407 | 0.933352679 | | -0.102612722 | 0.584957601 | |
| **395** | 0.053436412 | 0.436429803 | | 0.015103561 | 0.283445429 | |
| **396** | 0.006466468 | 0.934104808 | | 0.007342101 | 0.312439686 | |
| **397** | 0.002509858 | 0.906746807 | | -0.004153236 | 0.441918434 | |
| **398** | 0.122038276 | 0.082472597 | + | 0.121454021 | 0.004367657 | + |
| **399** | 0.0122082 | 0.852208748 | | -0.041331436 | 0.908741174 | |
| **400** | -0.072434611 | 0.363328485 | | -0.186232134 | 0.168615074 | |
| **401** | 0.080929641 | 0.136528011 | | 0.02645632 | 0.302705041 | |
| **402** | 0.049961783 | 0.454653388 | | 0.05502783 | 0.053440265 | + |
| **403** | 0.012977226 | 0.915127779 | | 0.024520377 | 0.557419299 | |
| **404** | 0.051752597 | 0.633418283 | | 0.01151041 | 0.426873611 | |
| **405** | 0.022948567 | 0.740398549 | | 0.033542171 | 0.152039517 | |
| **406** | -0.042060199 | 0.597964795 | | 0.016792373 | 0.278456276 | |
| **407** | 0.053610039 | 0.436057266 | | 0.059851423 | 0.067900449 | + |
| **408** | -0.020280744 | 0.820648771 | | -0.034967145 | 0.753207356 | |
| **409** | 0.023391857 | 0.781765718 | | -0.020024029 | 0.605793677 | |
| **410** | 0.041468438 | 0.562737565 | | 0.019923374 | 0.315419674 | |
| **411** | 0.049145035 | 0.601639514 | | 0.036347779 | 0.13932829 | |
| **412** | 0.024833019 | 0.64626033 | | -0.051746227 | 0.76903744 | |

**Table 2: A consolidated list of activating or inactivating residue mutations from table 1.**

| Residues identified with an * denote the known ligand binding positions identified in Figure 5. | | |
|---|---|---|
| **Position** | **Nb80 direction** | **BArr direction** |
| **6** | NA | Activating |
| **14** | NA | Activating |
| **15** | Activating | Activating |
| **17** | Activating | NA |
| **20** | Activating | NA |
| **24** | NA | Activating |
| **26** | Activating | Activating |
| **28** | NA | Activating |
| **36** | Activating | NA |
| **37** | Activating | Activating |
| **43** | Activating | NA |
| **44** | NA | Activating |
| **49** | NA | Activating |
| **50** | NA | Activating |
| **54** | NA | Inactivating |
| **64** | Activating | NA |
| **67** | Inactivating | NA |
| **71** | NA | Inactivating |
| **75** | NA | Inactivating |
| **76** | Inactivating | NA |
| **78** | NA | Inactivating |
| **79** | Inactivating | Inactivating |
| **80** | NA | Inactivating |
| **82** | Inactivating | Inactivating |
| **85** | Activating | NA |
| **87** | Activating | Activating |
| **91** | Activating | NA |
| **94** | NA | Activating |
| **95** | Activating | Activating |
| **96** | Activating | NA |
| **99** | NA | Inactivating |
| **101** | Activating | Activating |
| **102** | Activating | NA |
| **105** | Activating | NA |
| **106** | Inactivating | Inactivating |
| **111** | NA | Inactivating |
| **113*** | Inactivating | Inactivating |
| **114** | Inactivating | Inactivating |
| **117*** | Inactivating | Inactivating |
| **118** | Inactivating | Inactivating |
| **119** | Inactivating | Inactivating |
| **121** | Inactivating | Inactivating |
| **124** | Activating | Activating |
| **126** | NA | Inactivating |
| **127** | Inactivating | Inactivating |
| **128** | Inactivating | Inactivating |
| **129** | Inactivating | Inactivating |
| **130** | NA | Activating |
| **131** | Inactivating | Inactivating |
| **138** | Inactivating | Inactivating |
| **139** | Inactivating | Inactivating |
| **141** | Inactivating | Inactivating |
| **145** | Inactivating | NA |
| **153** | NA | Inactivating |
| **160** | NA | Inactivating |
| **161** | Inactivating | Inactivating |
| **164** | Inactivating | Inactivating |
| **165** | Inactivating | Inactivating |
| **166** | NA | Inactivating |
| **168** | Activating | NA |
| **174** | Activating | NA |
| **181** | NA | Inactivating |
| **182** | NA | Activating |
| **184** | Inactivating | Inactivating |
| **185** | Activating | NA |
| **187** | Activating | Activating |
| **190** | Inactivating | Inactivating |
| **191** | Inactivating | Inactivating |
| **193*** | Inactivating | Inactivating |
| **199** | Inactivating | Inactivating |
| **200** | NA | Inactivating |
| **203*** | Inactivating | Inactivating |
| **204** | Inactivating | Inactivating |
| **207*** | Inactivating | Inactivating |
| **208** | Inactivating | Inactivating |
| **209** | Inactivating | Inactivating |
| **211** | NA | Inactivating |
| **218** | Activating | Activating |
| **219** | Inactivating | Inactivating |
| **225** | NA | Activating |
| **229** | NA | Activating |
| **234** | Activating | NA |
| **236** | Activating | Activating |
| **240** | NA | Activating |
| **246** | NA | Activating |
| **255** | NA | Activating |
| **268** | Activating | Activating |
| **269** | Activating | NA |
| **272** | Activating | Activating |
| **276** | Activating | Activating |
| **277** | NA | Activating |
| **278** | Inactivating | Inactivating |
| **280** | Inactivating | Inactivating |
| **283** | Activating | Activating |
| **285** | Activating | NA |
| **286** | Inactivating | Inactivating |
| **289** | Inactivating | Inactivating |
| **290*** | Inactivating | Inactivating |
| **293*** | Inactivating | Inactivating |
| **294** | Inactivating | Inactivating |
| **295** | Activating | NA |
| **308** | NA | Inactivating |
| **312*** | Inactivating | Inactivating |
| **313** | Activating | Activating |
| **314** | NA | Activating |
| **315** | Inactivating | Inactivating |
| **316** | NA | Inactivating |
| **318** | Inactivating | Inactivating |
| **322** | Inactivating | Inactivating |
| **323** | Activating | Activating |
| **325** | Inactivating | Inactivating |
| **326** | Inactivating | Inactivating |
| **327** | Inactivating | Inactivating |
| **328** | Inactivating | Inactivating |
| **329** | Inactivating | Inactivating |
| **330** | NA | Inactivating |
| **346** | NA | Activating |
| **351** | NA | Activating |
| **352** | Activating | NA |
| **355** | NA | Activating |
| **357** | NA | Activating |
| **360** | NA | Activating |
| **362** | NA | Activating |
| **368** | NA | Activating |
| **369** | NA | Activating |
| **372** | NA | Activating |
| **373** | NA | Activating |
| **374** | NA | Activating |
| **375** | Activating | Activating |
| **383** | NA | Activating |
| **386** | NA | Activating |
| **388** | NA | Activating |
| **398** | Activating | Activating |
| **402** | NA | Activating |
| **407** | NA | Activating |

The above analysis is an example of how residues important for signalling can be discovered. An ongoing/future set of analyses will seek to find residues/pockets that are MORE important for signalling through one pathway versus the other (G protein or B-arrestin). This analysis will use data generated from the balanced ligand versus the biased ligand, and G-protein transducer versus B-arrestin transducer, to identify biased signalling hotspots.

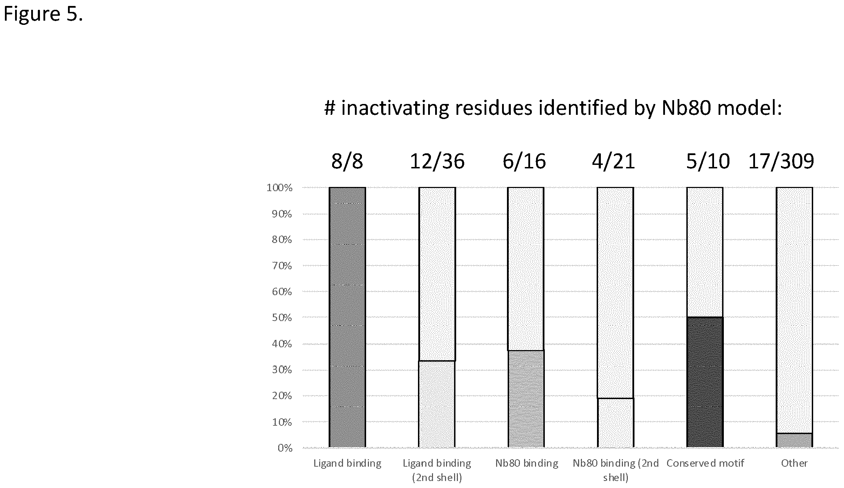

There is no existing list of known inactivating (or activating) residues. The data in figure 5 shows that categories of residues that could be expected to be inactivating are indeed enriched compared to the rest of the receptor ("Other"). The ligand binding residues are considered to be the strongest positive control (under the assumption that mutating a residue that forms a molecular contact with the ligand should disrupt ligand binding and therefore receptor activation).

G-proteins are difficult to work with in assays because they are heterotrimers and because the Go subunit (which is the subunit responsible for binding the GPCR) is membrane tethered. Therefore, it is difficult to make fusion constructs, and the membrane tethering results in a high background signal. "mini-G" proteins have been developed that are monomeric and cytosolic. A major focus of future development for this project would be to validate / optimize the use of miniG proteins.

There are four families of Go proteins (Gₛ, G_{i/o}, G_{q}, and G_{12/13}) and a "miniG" representative exists for each class. Some GPCRs signal through only one G-protein while others signal through two or more. MiniGs work with the protocol/methods as presently described, albeit with reduced signal:noise compared with B-arrestin or Nb80. Hence, such methods are encompassed, which enable multiple readouts that would empower detection of mutations that bias not only between B-arrestin and G-protein, but actually mutations that bias between different G-proteins.

### NUMBERED EMBODIMENTS

**The following numbered paragraphs relate to the method for identifying target sites:**
1. A method for identifying one or more target sites of a G-protein coupled receptor (GPCR), the method comprising:
   obtaining a training data set specifying, for each of a plurality of variants of the GPCR having different combinations of one or more mutations, a surface expression measure and a functional measure for that variant, the functional measure quantifying an extent to which the variant is functional for a given purpose;
   fitting a model to the training data set, to obtain a set of model parameters indicative of a correlation between the surface expression measure and the functional measure for the variants of the GPCR; and
   based on the set of model parameters obtained by fitting the model, identifying the one or more target sites of the GPCR.
2. The method of any preceding paragraph, in which the one or more target sites are identified based on residuals indicating, for each variant of the GPCR, a difference between the functional measure and a predicted functional measure predicted from the surface expression measure based on the set of model parameters.
3. The method of paragraph 2, in which identifying the one or more target sites comprises ranking the target sites based on an aggregate measure associated with each candidate site, the aggregate measure for a given candidate site being derived from the residuals associated with variants having a mutation at the given candidate site.
4. The method of paragraph 3, in which the aggregate measure comprises a mean, median, minimum or maximum of the residuals associated with variants having a mutation at the given candidate site.
5. The method of paragraph 2, in which identifying the one or more target sites comprises determining, for each candidate site, a score value based on applying a statistical test to a group of residuals corresponding to variants having a mutation at that candidate site, and selecting the one or more target sites based on the score values determined for each candidate site.
6. The method of paragraph 5, in which the score value is indicative of whether the group of residuals indicates that it is statistically likely that the candidate site has an effect on the target property of interest.
7. The method of any preceding paragraph, in which the model comprises a regression model.
8. The method of any preceding paragraph, in which the model comprises a local regression model.
9. The method of any preceding paragraph, in which the model comprises a Loess regression model.
10. The method of any preceding paragraph, comprising fitting a model to each of a plurality of training data sets based on two or more different functional measures, and identifying the one or more target sites based on a plurality of sets of model parameters obtained by fitting the model to the plurality of training data sets.
11. The method of any preceding paragraph, comprising:
   obtaining a first training data set specifying, for the plurality of variants of the GPCR, a surface expression measure and a first functional measure for that variant;
   obtaining a second training data set specifying, for the plurality of variants of the GPCR, the surface expression measure and a second functional measure for that variant;
   fitting the model to the first training data set to obtain a first set of model parameters;
   fitting the model to the second training data set to obtain a second set of model parameters; and
   identifying the one or more target sites based on the first set of model parameters and the second set of model parameters.
12. The method of paragraph 11, in which the one or more target sites comprise one or more target sites for which selection criteria are satisfied based on both the first set of model parameters and the second set of model parameters.
13. The method of paragraph 11 in which the one or more target sites comprise one or more target sites for which selection criteria are satisfied based on either one of the first set of model parameters and the second set of model parameters.
14. The method of paragraph 11, in which the one or more target sites comprise one or more target sites for which selection criteria are satisfied based on one of the first set of model parameters and the second set of model parameters and the selection criteria are not satisfied based on the other of the first set of model parameters and the second set of model parameters.
15. The method of any preceding paragraph, wherein the surface expression measure is a measurement obtained using an assay that comprises quantification and/or separation of cells expressing the GPCR variant, wherein the GPCR variant is labelled.
16. The method of paragraph 15, wherein in the assay for obtaining the surface expression measure, the GPCR is fluorescently labelled, optionally using a conjugated antibody or variant thereof.
17. The method of any of paragraphs 15 and 16, wherein the quantification and/or separation is achieved using fluorescence-activated cell sorting (FACS).
18. The method of any preceding paragraph, in which, for a given variant of the GPCR:
   the surface expression measure is a first surface expression measure indicative of surface expression of the given variant of the GPCR in absence of a ligand; and
   the functional measure is a second surface expression measure indicative of surface expression of the given variant of the GPCR in presence of a ligand.
19. The method of paragraph 18, wherein the ligand is:
   (i) a balanced agonist;
   (ii) a biased agonist;
   (iii) a balanced antagonist; or
   (iv) a biased antagonist.
20. The method of paragraph 18 or paragraph 19, wherein the ligand increases the expression of the variant of the GPCR at the cell surface.
21. The method of paragraph 18 or paragraph 19, wherein the ligand decreases the expression of the variant of the GPCR at the cell surface.
22. The method of any one of paragraphs 18-21, wherein the ligand is a known ligand of the GPCR.
23. The method of any one of paragraphs 18-22, wherein the ligand has an unknown binding site on the GPCR.
24. The method of any of paragraphs 1 to 17, in which the functional measure is a signalling measure.
25. The method of paragraph 24, wherein the signalling measure is a receptor-proximal measure.
26. The method of any of paragraphs 24 and 25, wherein the signalling measure is obtained using an assay that does not rely upon endogenous cellular secondary messengers, optionally wherein said secondary messenger is cAMP.
27. The method of any of paragraphs 24 to 26, wherein the signalling measure is obtained using an assay wherein:
   (i) recruitment of a first intracellular signalling component to the GPCR results in the liberation of a transcription activating component; and
   (ii) the transcription activating component induces transcription of a reporter gene.
28. The method of any of paragraphs 24 to 27, wherein liberation of a transcription activating component is achieved by enzymatic cleavage of the transcription activating component from the GPCR by the first intracellular signalling component.
29. The method of any of paragraphs 24 to 28, wherein:
   (i) the first intracellular signalling component is a G-protein or variant thereof that signals via a G-protein dependent pathway;
   (ii) the first intracellular signalling component is β-arrestin or variant thereof that signals via a β-arrestin dependent pathway;
   (iii) the transcription activating component is a transcription factor of variant thereof, optionally Gal4; and/or
   (iv) the reporter gene comprises a barcode that is unique to a specific variant of the GPCR.
30. The method of any of paragraphs 24 to 29, wherein the signalling measure is a measurement relating to signalling in a:
   (i) pathway that is instigated by or involves the activation of a GPCR;
   (ii) a G-protein mediated pathway; and/or
   (iii) a β-arrestin mediated pathway.
31. The method of any of paragraphs 24 to 30, comprising fitting a model to a plurality of training data sets based on two or more different signalling measures, and identifying the one or more target sites based on the model parameters obtained by fitting the model to the plurality of training data sets.
32. The method of any of paragraphs 24 to 31, comprising:
   obtaining a first training data set specifying, for the plurality of variants of the GPCR, a surface expression measure and a first signalling measure for that variant;
   obtaining a second training data set specifying, for the plurality of variants of the GPCR, the surface expression measure and a second signalling measure for that variant;
   fitting the model to the first training data set to obtain a first set of model parameters;
   fitting the model to the second training data set to obtain a second set of model parameters; and
   identifying the one or more target sites based on the first set of model parameters and the second set of model parameters.
33. The method of any of paragraphs 31 to 32, wherein the two or more different signalling measures comprise signalling measures relating to two or more signalling pathways.
34. The method of paragraph 33, wherein one of the two or more signalling pathways is a G-protein mediated pathway.
35. The method of any of paragraphs 33 and 34, wherein one of the two or more signalling pathways is a β-arrestin mediated pathway.
36. The method of any of paragraphs 31 and 32, wherein the two or more different signalling measures comprise signalling measures depending on signalling in response to different ligands.
37. The method of paragraph 36, wherein the first and/or second ligand is selected from the group consisting of:
   (i) a balanced agonist;
   (ii) a biased agonist;
   (iii) a balanced antagonist; and
   (iv) a biased antagonist.
38. The method of any one of paragraphs 18-23 and 36-37, wherein ligand is an endogenous ligand or an exogenous ligand.
39. The method of paragraph 19 or paragraph 37, wherein the biased agonist or the biased antagonist is an agonist/antagonist of a G-protein mediated pathway or a β-arrestin mediated pathway.
40. The method of paragraph 19 or 37, wherein the balanced agonist or the balanced antagonist is an agonist/antagonist of a G-protein mediated pathway and a β-arrestin mediated pathway.
41. A computer program to control a computer to perform the method of any preceding paragraph.
42. A computer-readable storage medium storing the computer program of paragraph 41.
43. An apparatus comprising processing circuitry configured to perform the method of any preceding paragraph.
44. A nucleic acid molecule or plurality of nucleic acid molecules encoding:
   (i) a receptor construct;
   (ii) a transducer construct; and/or
   (iii) a reporter construct.
45. The nucleic acid molecule or plurality of nucleic acid molecules of paragraph 44, wherein:
   (i) the receptor construct comprises a sequence encoding a variant of a GPCR, wherein said variant is fused to a stimulus sensing component, and a transcription activating component, and wherein said construct comprises a protease cleavage site between said sensing and transcription activating components;
   (ii) the transducer construct comprises a sequence encoding a first intracellular signalling component that is fused to a stimulus generating component and a protease component, wherein said protease is able to cleave at the protease cleavage site; and/or
   (iii) the reporter construct comprises a sequence encoding a sequence that is subject to transcriptional regulation by the transcription activating component, a reporter gene, and a barcode sequence.
46. The nucleic acid molecule or plurality of nucleic acid molecules of paragraph 45, wherein:
   (i) the heterologous stimulus sensing component is a Light, Oxygen, Voltage sensitive (LOV) domain (LOV);
   (ii) the transcription activating component is Gal4;
   (iii) the protease cleavage site is a TEV cleavage site;
   (iv) the first intracellular signalling component is:
      a. a G-protein or variant thereof;
      b. β-arrestin or a variant thereof; or
      c. a conformation-specific GPCR-binding protein, optionally a nanobody;
   (v) the stimulus generating component is a NanoLuciferase;
   (vi) the protease component is a TEV protease;
   (vii) the sequence that is subject to transcriptional regulation by the transcription activating component is a Gal4 upstream activator sequence (UAS);
   (viii) the reporter gene is a fluorescent protein, optionally citrine;
   (ix) wherein the sequence that is subject to transcriptional regulation, the reporter gene, and the barcode sequence are operably linked; and/or
   (x) the barcode sequence is a barcode sequence that is unique to a specific variant of a GPCR, optionally wherein said barcode sequence is present in the 3'UTR of the reporter construct.
47. The nucleic acid molecule or plurality of nucleic acid molecules of any one of paragraphs 44-46, wherein:
   (i) the receptor construct, the transducer construct, and the reporter construct are encoded in different nucleic acid molecules;
   (ii) the receptor construct, the transducer construct, and the reporter construct are encoded in the same nucleic acid molecule;
   (iii) two of the receptor construct, the transducer construct, and/or the reporter construct are encoded in the same nucleic acid molecule, and the remaining is encoded in a different nucleic acid molecule.
48. The nucleic acid molecule or plurality of nucleic acid molecules of paragraph47, wherein the receptor construct and the reporter construct are encoded in the same nucleic acid molecule.
49. A vector or plurality of vectors encoding the nucleic acid molecule or plurality of nucleic acid molecules of any one of paragraphs 44-48.
50. A cell comprising the nucleic acid molecule or plurality of nucleic acid molecules of any one of paragraphs 44-48, or the vector or plurality of vectors of paragraph 49.
51. The cell of paragraph 50, wherein the cell comprises one or more nucleic acid molecules or one or more vectors encoding the receptor construct and the reporter construct.

**The following numbered paragraphs relate to a method of modulating the activity of a β₂-adrenergic receptor (β₂AR) and related aspects of the invention:**
1. A method of modulating the activity of a β₂-adrenergic receptor (β₂AR), the method comprising the step of mutating one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, 1182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.
2. The method of paragraph 1, wherein one, two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, thirty one, thirty two, thirty three, thirty four, thirty five, thirty six, thirty seven, thirty eight, thirty nine, forty, forty one, forty two, forty three, forty four, forty five, forty six, forty seven, forty eight, forty nine, fifty, fifty one, fifty two, fifty three, fifty four, fifty five, fifty six, fifty seven, fifty eight, fifty nine, sixty, sixty one, sixty two, sixty three, sixty four, sixty five, sixty six, sixty seven, sixty eight, sixty nine, seventy, seventy one, seventy two, seventy three, seventy four, seventy five, seventy six, seventy seven, seventy eight, seventy nine, eighty, eighty one, eighty two, eighty three, eighty four, eighty five, eighty six, eighty seven, eighty eight, eighty nine, ninety, ninety one, ninety two, ninety three, ninety four, ninety five, ninety six, ninety seven, ninety eight, ninety nine, one hundred, one hundred and one, one hundred and two, one hundred and three, one hundred and four, one hundred and five, one hundred and six, one hundred and seven, one hundred and eight, one hundred and nine, one hundred and ten, one hundred and eleven, one hundred and twelve, one hundred and thirteen, one hundred and fourteen, one hundred and fifteen, one hundred and sixteen, one hundred and seventeen, one hundred and eighteen, one hundred and nineteen, one hundred and twenty, one hundred and twenty one, one hundred and twenty two, one hundred and twenty three, one hundred and twenty four, one hundred and twenty five, one hundred and twenty six, one hundred and twenty seven, one hundred and twenty eight, one hundred and twenty nine, one hundred and thirty, one hundred and thirty one, one hundred and thirty two, one hundred and thirty three, one hundred and thirty four, or one hundred and thirty five residues are mutated.
3. The method of paragraph 1 or paragraph 2, wherein the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.
4. The method of any one of paragraphs 1 to 3, wherein the one or more residues is selected from the group consisting of: D79, M82, C106, D113, V114, V117, T118, A119, 1121, 1127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.
5. The method of any one of paragraphs 1 to 3, wherein the one or more residues is selected from the group consisting of: V67, A76, and L145.
6. The method of any one of paragraphs 1 to 3, wherein the one or more residues is selected from the group consisting of: V54, F71, L75, A78, L80, W99, S111, V126, I153, V160, F166, A181, A200, P211, Y308, Y316, and P330.
7. The method of paragraph 1 or paragraph 2, wherein the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, 1182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407.
8. The method of paragraph 1, paragraph 2 or paragraph 7, wherein the one or more residues is selected from the group consisting of: N15, Q26, G37, V87, L95, F101, L124, N187, V218, S236, E268, L272, G276, T283, W313, P323, K375, and N398.
9. The method of paragraph 1, paragraph 2 or paragraph 7, wherein the one or more residues is selected from the group consisting of: S17, P20, M36, I43, L64, A85, A91, M96, G102, W105, P168, Y174, Y185, D234, H269, C285, V295, and N352.
10. The method of paragraph 1, paragraph 2 or paragraph 7, wherein the one or more residues is selected from the group consisting of: N6, P14, V24, R28, V44, F49, G50, I94, D130, 1182, E225, Q229, F240, S246, G255, I277, I314, S346, G351, S355, N357, T360, E362, V368, E369, K372, E373, N374, G383, D386, V388, Q402, and S407.
11. The method of any one of the preceding paragraphs, wherein the one or more residues is located within an allosteric site.
12. The method of any one of the preceding paragraphs, wherein the modulating is activating or inactivating.
13. The method of paragraph 12, wherein the modulating is activating.
14. The method of paragraph 12, wherein the modulating is inactivating.
15. A binding molecule which binds to one or more target sites on a β₂-adrenergic receptor (β₂AR), wherein the one or more target sites comprises one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, 1153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.
16. The binding molecule of paragraph 15, wherein the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, I182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407.
17. The binding molecule of paragraph 15 or paragraph 16, wherein the one or more residues is selected from the group consisting of: N15, Q26, G37, V87, L95, F101, L124, N187, V218, S236, E268, L272, G276, T283, W313, P323, K375, and N398.
18. The binding molecule of paragraph 15 or paragraph 16, wherein the one or more residues is selected from the group consisting of: S17, P20, M36, I43, L64, A85, A91, M96, G102, W105, P168, Y174, Y185, D234, H269, C285, V295, and N352.
19. The binding molecule of paragraph 15 or paragraph 16, wherein the one or more residues is selected from the group consisting of: N6, P14, V24, R28, V44, F49, G50, I94, D130, 1182, E225, Q229, F240, S246, G255, I277, I314, S346, G351, S355, N357, T360, E362, V368, E369, K372, E373, N374, G383, D386, V388, Q402, and S407.
20. The binding molecule of paragraph 15, wherein the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.
21. The binding molecule of paragraph 15 or paragraph 20, wherein the one or more residues is selected from the group consisting of: D79, M82, C106, D113, V114, V117, T118, A119, I121, I127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.
22. The binding molecule of paragraph 15 or paragraph 20, wherein the one or more residues is selected from the group consisting of: V67, A76, and L145.
23. The binding molecule of paragraph 15 or paragraph 20, wherein the one or more residues is selected from the group consisting of: V54, F71, L75, A78, L80, W99, S111, V126, I153, V160, F166, A181, A200, P211, Y308, Y316, and P330.
24. The binding molecule of any one of paragraphs 15 to 23, wherein the one or more target sites is druggable.
25. The binding molecule of any one of paragraphs 15 to 24, wherein the binding molecule is:
   (i) a polypeptide;
   (ii) a nucleic acid; or
   (iii) a small molecule.
26. The binding molecule of any one of paragraphs 15 to 25, wherein the binding molecule is an antibody, or a mimetic or derivative thereof, optionally an affibody, a nanobody, a Fab fragment, or a scFv.
27. The binding molecule of any one of paragraphs 15 to 26, wherein the binding molecule modulates the activity of β₂AR, optionally wherein the modulating is an increase or a decrease in activity.
28. The binding molecule of paragraph 27, wherein the activity of β₂AR is the activity on:
   (i) a G-protein; and/or
   (ii) β-arrestin.
29. A polypeptide encoding a β₂-adrenergic receptor (β₂AR) variant, wherein the polypeptide comprises a mutation, relative to a wild-type β₂AR, at one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, 1153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.
30. The polypeptide of paragraph 29, wherein the one or more residues is selected from the group consisting of: N6, P14, N15, S17, P20, V24, Q26, R28, M36, G37, I43, V44, F49, G50, L64, A85, V87, A91, I94, L95, M96, F101, G102, W105, L124, D130, P168, Y174, I182, Y185, N187, V218, E225, Q229, D234, S236, F240, S246, G255, E268, H269, L272, G276, I277, T283, C285, V295, W313, I314, P323, S346, G351, N352, S355, N357, T360, E362, V368, E369, K372, E373, N374, K375, G383, D386, V388, N398, Q402, and S407..
31. The polypeptide of paragraph 29 or paragraph 30, wherein the one or more residues is selected from the group consisting of: N15, Q26, G37, V87, L95, F101, L124, N187, V218, S236, E268, L272, G276, T283, W313, P323, K375, and N398.
32. The polypeptide of paragraph 29 or paragraph 30, wherein the one or more residues is selected from the group consisting of: S17, P20, M36, I43, L64, A85, A91, M96, G102, W105, P168, Y174, Y185, D234, H269, C285, V295, and N352.
33. The polypeptide of paragraph 29 or paragraph 30, wherein the one or more residues is selected from the group consisting of: N6, P14, V24, R28, V44, F49, G50, I94, D130, I182, E225, Q229, F240, S246, G255, I277, I314, S346, G351, S355, N357, T360, E362, V368, E369, K372, E373, N374, G383, D386, V388, Q402, and S407.
34. The polypeptide of paragraph 29, wherein the one or more residues is selected from the group consisting of: V54, V67, F71, L75, A76, A78, D79, L80, M82, W99, C106, S111, D113, V114, V117, T118, A119, I121, V126, I127, A128, V129, R131, P138, F139, Y141, L145, I153, V160, S161, T164, S165, F166, A181, C184, C190, C191, F193, Y199, A200, S203, S204, S207, F208, Y209, P211, Y219, I278, G280, W286, F289, F290, N293, I294, Y308, N312, G315, Y316, N318, N322, I325, Y326, C327, R328, S329, and P330.
35. The polypeptide of paragraph 29 or paragraph 34, wherein the one or more residues is selected from the group consisting of: D79, M82, C106, D113, V114, V117, T118, A119, 1121, 1127, A128, V129, R131, P138, F139, Y141, S161, T164, S165, C184, C190, C191, F193, Y199, S203, S204, S207, F208, Y209, Y219, I278, G280, W286, F289, F290, N293, I294, N312, G315, N318, N322, I325, Y326, C327, R328, and S329.
36. The polypeptide of paragraph 29 or paragraph 34, wherein the one or more residues is selected from the group consisting of: V67, A76, and L145.
37. The polypeptide of paragraph 29 or paragraph 34, wherein the one or more residues is selected from the group consisting of: V54, F71, L75, A78, L80, W99, S111, V126, I153, V160, F166, A181, A200, P211, Y308, Y316, and P330.
38. The polypeptide of any one of paragraphs 29 to 37, wherein the one or more residues is located within an allosteric site.
39. The polypeptide of any one of paragraphs 29 to 38, wherein the activity of the polypeptide is modulated relative to the activity of a wild-type β₂AR, optionally wherein the modulated activity is an increase or a decrease in activity.
40. The polypeptide of paragraph 39, wherein the activity is the activity on:
   (i) a G-protein; and/or
   (ii) β-arrestin.
41. A nucleic acid encoding:
   (i) the binding molecule of any one of paragraphs 15 to 28; and/or
   (ii) the polypeptide of any one of paragraphs 29 to 40.
42. The nucleic acid of paragraph 41, wherein the nucleic acid is RNA or DNA, optionally wherein the nucleic acid is modified, unmodified, naturally occurring or synthetic.
43. An expression cassette comprising the nucleic acid of paragraph 41 or paragraph 42.
44. A vector comprising the nucleic acid of paragraph 41 or paragraph 42, or the expression cassette of paragraph 43.
45. A cell comprising the binding molecule of any one of paragraphs 15 to 28, the polypeptide of any one of paragraphs 29 to 40, the nucleic acid of paragraph 41 or paragraph 42, the expression cassette of paragraph 43, or the vector of paragraph 44.
46. The binding molecule of any one of paragraphs 15 to 28, the polypeptide of any one of paragraphs 29 to 40, the nucleic acid of paragraph 41 or paragraph 42, the expression cassette of paragraph 43, the vector of paragraph 44, or the cell of paragraph 45, for use in a method of treating a disease.
47. A method of treating a disease comprising administering the binding molecule of any one of paragraphs 15 to 28, the polypeptide of any one of paragraphs 29 to 40, the nucleic acid of paragraph 41 or paragraph 42, the expression cassette of paragraph 43, the vector of paragraph 44, or the cell of paragraph 45, to a patient in need thereof.
48. Use of the binding molecule of any one of paragraphs 15 to 28, the polypeptide of any one of paragraphs 29 to 40, the nucleic acid of paragraph 41 or paragraph 42, the expression cassette of paragraph 43, the vector of paragraph 44, or the cell of paragraph 45, for the manufacture of a medicament for use in the treatment of a disease.
49. The binding molecule, polypeptide, nucleic acid, expression cassette, vector, or cell, for use of paragraph 46, the method of paragraph 47, or the use of paragraph 48, wherein the disease is:
   (i) a neurological disease or disorder;
   (ii) a cardiovascular disease or disorder;
   (iii) a respiratory disease or disorder;
   (iv) a musculoskeletal disease or disorder;
   (v) a renal disease or disorder;
   (vi) an immune disease or disorder; and/or
   (vii) cancer.
50. The binding molecule, polypeptide, nucleic acid, expression cassette, vector, or cell, for use, the method or the use of paragraph 49, wherein:
   (i) the respiratory disease or disorder is selected from the group consisting of:
      asthma, chronic obstructive pulmonary disease (COPD), bronchospasm caused by COPD, bronchial asthma, and emphysema;
   (ii) the immune disease or disorder is selected from the group consisting of:
      rheumatoid arthritis (RA), systemic lupus erythematosus (SLE), multiple sclerosis (MS), myasthenia gravis (MG), and Grave's disease;
   (iii) the cancer is selected from the group consisting of: lung cancer, breast cancer, prostate cancer, and skin cancer; and
   (iv) the cardiovascular disease or disorder is hyperkalemia, or vascular extravasation.

## Claims

1. A method of modulating the activity of a β₂-adrenergic receptor (β₂AR), the method comprising the step of mutating one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, I153, V160, S161, T164, S165, F166, P168, Y174, A181, 1182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407

2. The method of claim 1, wherein the modulating is activating or inactivating.

3. A binding molecule which binds to one or more target sites on a β₂-adrenergic receptor (β₂AR), wherein the one or more target sites comprises one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, 1153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

4. The binding molecule of claim 3, wherein the one or more target sites is druggable.

5. The binding molecule of claim 3 or claim 4, wherein the binding molecule is:
(i) a polypeptide;
(ii) a nucleic acid; or
(iii) a small molecule.

6. The binding molecule of any one of claims 3 to 5, wherein the binding molecule is an antibody, or a mimetic or derivative thereof, optionally an affibody, a nanobody, a Fab fragment, or a scFv.

7. The binding molecule of any one of claims 3 to 6, wherein the binding molecule modulates the activity of β₂AR, optionally wherein the modulating is an increase or a decrease in activity.

8. A polypeptide encoding a β₂-adrenergic receptor (β₂AR) variant, wherein the polypeptide comprises a mutation, relative to a wild-type β₂AR, at one or more residues selected from the group consisting of: N6, P14, N15, S17, P20, V24, Q26, R28, M36, G37, I43, V44, F49, G50, V54, L64, V67, F71, L75, A76, A78, D79, L80, M82, A85, V87, A91, I94, L95, M96, W99, F101, G102, W105, C106, S111, D113, V114, V117, T118, A119, I121, L124, V126, I127, A128, V129, D130, R131, P138, F139, Y141, L145, 1153, V160, S161, T164, S165, F166, P168, Y174, A181, I182, C184, Y185, N187, C190, C191, F193, Y199, A200, S203, S204, S207, F208, Y209, P211, V218, Y219, E225, Q229, D234, S236, F240, S246, G255, E268, H269, L272, G276, I277, I278, G280, T283, C285, W286, F289, F290, N293, I294, V295, Y308, N312, W313, I314, G315, Y316, N318, N322, P323, I325, Y326, C327, R328, S329, P330, S346, G351, N352, S355, N357, T360, E362, V368, E369, K372, E373, N374, K375, G383, D386, V388, N398, Q402, and S407.

9. A nucleic acid encoding:
(i) the binding molecule of any one of claims 3 to 7; and/or
(ii) the polypeptide of claim 8.

10. The nucleic acid of claim 9, wherein the nucleic acid is RNA or DNA, optionally wherein the nucleic acid is modified, unmodified, naturally occurring or synthetic.

11. An expression cassette comprising the nucleic acid of claim 9 or claim 10.

12. A vector comprising the nucleic acid of claim 9 or claim 10, or the expression cassette of claim 11.

13. A cell comprising the binding molecule of any one of claims 3 to 7, the polypeptide of claim 8, the nucleic acid of claim 9 or claim 10, the expression cassette of claim 11, or the vector of claim 12.

14. The binding molecule of any one of claims 3 to 7, the polypeptide of claim 8, the nucleic acid of claim 9 or claim 10, the expression cassette of claim 11, the vector of claim 12, or the cell of claim 13, for use in a method of treating a disease.

15. The binding molecule, polypeptide, nucleic acid, expression cassette, vector, or cell, for use of claim 14, wherein the disease is:
(i) a neurological disease or disorder;
(ii) a cardiovascular disease or disorder;
(iii) a respiratory disease or disorder;
(iv) a musculoskeletal disease or disorder;
(v) a renal disease or disorder;
(vi) an immune disease or disorder; and/or
(vii) cancer.
